(19) Europäisches Patentamt
European Patent Office
Office européen des brevets

(11) **EP 4 101 839 A1**

(12) **EUROPEAN PATENT APPLICATION**
published in accordance with Art. 153(4) EPC


(43) Date of publication:
**14.12.2022 Bulletin 2022/50**

(21) Application number: **21752925.4**

(22) Date of filing: **12.02.2021**

(51) International Patent Classification (IPC):
***C07C 229/36*** $^{(2006.01)}$ ***A61K 31/198*** $^{(2006.01)}$
***A61K 31/223*** $^{(2006.01)}$ ***A61P 9/00*** $^{(2006.01)}$
***A61P 11/00*** $^{(2006.01)}$ ***A61P 25/00*** $^{(2006.01)}$
***A61P 31/04*** $^{(2006.01)}$ ***A61P 39/06*** $^{(2006.01)}$
***A61P 43/00*** $^{(2006.01)}$

(52) Cooperative Patent Classification (CPC):
**C07C 229/36; A61K 31/198; A61K 31/223; A61P 9/00; A61P 11/00; A61P 25/00; A61P 31/04; A61P 39/06; A61P 43/00**

(86) International application number:
**PCT/JP2021/005302**

(87) International publication number:
**WO 2021/162103 (19.08.2021 Gazette 2021/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.02.2020 JP 2020023164**
**14.02.2020 JP 2020023221**



(71) Applicants:
- **Feliqs Corporation**
**Fukuoka-shi, Fukuoka 812-0044 (JP)**
- **Kyushu University, National University Corporation**
**Nishi-ku**
**Fukuoka-shi**
**Fukuoka 819-0395 (JP)**

(72) Inventors:
- **YAMADA Kenichi**
**Fukuoka-shi, Fukuoka 819-0395 (JP)**
- **KUNINOBU Kenichiro**
**Fukuoka-shi, Fukuoka 812-0044 (JP)**

(74) Representative: **J A Kemp LLP**
**80 Turnmill Street**
**London EC1M 5QU (GB)**


(54) **COMPOUND AND USE THEREOF**


(57) Provided is a compound that achieves a good balance between the LO• scavenging ability and the LOO• scavenging ability. The compound of the present invention or a salt thereof is represented by the following formula (1). In the formula (1), $R^1$ and $R^2$ may be the same or different and are each independently a hydrogen atom or an alkyl group, $R^3$ is $-OR^4$ or $-NHR^5$, $R^4$ is a sec-butyl group, a tert-butyl group, or an iso-butyl group, and $R^5$ is a sec-butyl group, a tert-butyl group, or an iso-butyl group.


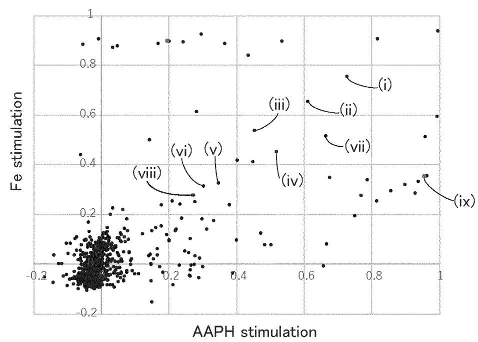


(i) Compound of formula (3)
(ii) Compound of formula (4)
(iii) Compound of formula (5)
(iv) Compound of formula (7)
(v) Compound of formula (8)
(vi) Compound of formula (9)
(vii) Compound offormula (10)
(viii) Edaravone
(ix) Methyldopa(L−isomer)

FIG. 4

EP 4 101 839 A1

## Description

TECHNICAL FIELD

The present invention relates to a compound and use thereof.

BACKGROUND ART

Diseases in which lipid radicals in lipid peroxidation are involved cover a wide variety of disease areas, including cardiovascular diseases, central nervous system diseases, respiratory system diseases, and diseases to which antimicrobial agents are applicable (Non-Patent Document 1). However, since a large number of lipid peroxides and metabolic products thereof are involved in each one of the diseases caused by lipid peroxidation, it is not at all easy to search for a useful therapeutic agent on a disease-specific basis. Moreover, although there are many known antioxidative substances, edaravone (5-methyl-2-phenyl-2,4-dihydro-3H-pyrazol-3-one, trade name RADICUT, Mitsubishi Tanabe Pharma Corporation) is the only antioxidative substance approved for use as a drug. Under these circumstances, there has been demand for a drug that exhibits a suppressing effect against lipid peroxidation.

Citation List

Non-Patent Literature

Non-Patent Literature 1: Jeroen Frijhoff et al., "Clinical Relevance of Biomarkers of Oxidative Stress," Antioxidants & Redox Signaling, Vol. 23, No. 14, pages 1144-1170

SUMMARY OF INVENTION

Technical Problems

In light of the foregoing, the inventors of the present invention found through in-depth studies that methyldopa (Aldomet®), which is an anti-hypertensive agent, can function as an antioxidant. However, the inventors of the present invention noted that, although methyldopa has a high ability (LOO• scavenging ability) to scavenge peroxyl radicals (LOO•), its ability (LO• scavenging ability) to scavenge alkoxyl radicals (LO•), which are known to be more reactive than LOO•, is low as compared with the LOO• scavenging ability.

The inventors also examined the LO• scavenging ability and the LOO• scavenging ability of edaravone. As a result, it was found that edaravone has approximately the same level of scavenging ability against LO• and LOO•. On this account, when an antioxidant is used as a drug, it is considered to be desirable that the antioxidant have a good balance between the LO• scavenging ability and the LOO• scavenging ability.

In light of the foregoing, it is an object of the present invention to provide a compound that is superior to methyldopa in terms of a balance between the LOO• scavenging ability and the LO• scavenging ability.

Solution to Problem

In order to achieve the above object, the present invention provides a compound represented by the following formula (1) or a salt thereof.

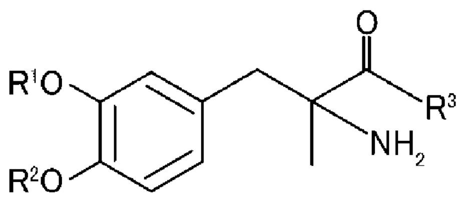

···(1)

In the formula (1),

$R^1$ and $R^2$ may be the same or different and are each independently a hydrogen atom, an alkyl group, or an acyl group,
$R^3$ is $-OR^4$ or $-NHR^5$,
$R^4$ is a sec-butyl group, a tert-butyl group, or an iso-butyl group, and

$R^5$ is a sec-butyl group, a tert-butyl group, or an iso-butyl group.

The present invention also provides a pharmaceutical composition containing the compound of the present invention or a salt thereof and a pharmaceutically acceptable carrier.

The present invention also provides an antioxidant containing a compound represented by the following formula (1A) or a salt thereof.

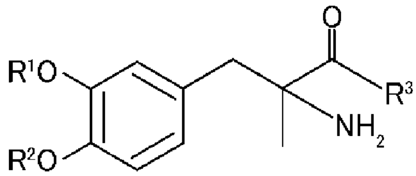

···(1A)

In the formula (1A),

$R^1$ and $R^2$ may be the same or different and are each independently a hydrogen atom, an alkyl group, or an acyl group,
$R^3$ is $-OR^4$ or $NHR^5$,
$R^4$ is an alkyl group, and
$R^5$ is a hydrogen atom or an alkyl group.

The present invention also provides a cell protectant containing the compound of the present invention or a salt thereof and the antioxidant of the present invention.

The present invention also provides a cell growth-promoting agent containing the compound of the present invention or a salt thereof and the antioxidant of the present invention.

The present invention also provides a method for preventing oxidation using the antioxidant of the present invention.

The present invention also provides a method for protecting a cell using the cell protectant of the present invention.

The present invention also provides a method for promoting cell growth using the cell growth-promoting agent of the present invention.

Advantageous Effects of Invention

The compound of the present invention or a salt thereof is superior to methyldopa in terms of achieving a balance between the LOO• scavenging ability and the LO• scavenging ability.

BRIEF DESCRIPTION OF DRAWINGS

[FIG. 1] FIG. 1 is a graph showing the result of NMR measurement on a compound (3) in Example 1.
[FIG. 2] FIG. 2 is a graph showing the result of NMR measurement on a compound (4) in Example 1.
[FIG. 3] FIG. 3 is a graph showing the result of NMR measurement on a compound (5) in Example 1.
[FIG. 4] FIG. 4 is a graph showing the LOO• scavenging abilities and the LO• scavenging abilities observed in Example 2.
[FIG. 5] FIG. 5 shows graphs showing the cell viability observed in Example 3.
[FIGs. 6A and 6B] FIGs. 6A and 6B are graphs showing the cell viability observed in Example 4.

DESCRIPTION OF EMBODIMENTS

Compound or Salt Thereof

As described above, the compound of the present invention or a salt thereof is represented by the following formula (1).

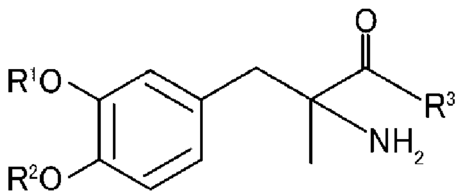

···(1)

In the formula (1),

- $R^1$ and $R^2$ may be the same or different and are each independently a hydrogen atom, an alkyl group, or an acyl group,
- $R^3$ is $-OR^4$ or $-NHR^5$,
- $R^4$ is a sec-butyl group, a tert-butyl group, or an iso-butyl group, and
- $R^5$ is a sec-butyl group, a tert-butyl group, or an iso-butyl group.

The compound of the present invention or a salt thereof is characterized in that it contains the compound represented by the formula (1) (hereinafter also referred to as "the compound of the present invention") or a salt thereof, and there is no particular limitation on other compositions and conditions. The compound of the present invention or a salt thereof is superior to methyldopa in terms of achieving a balance between the LOO• scavenging ability and the LO• scavenging ability. Accordingly, the compound of the present invention or a salt thereof is presumed to function as an excellent antioxidant in living organisms. Unless otherwise stated, the following description regarding the compound of the present invention also applies to salts of the compound of the present invention and to solvates of the compound of the present invention or a salt thereof.

The inventors of the present invention conducted in-depth studies to obtain compounds that are superior to methyldopa in terms of achieving a balance between the LOO• scavenging ability and the LO• scavenging ability. As a result, they discovered that compounds obtained by esterifying a carboxyl group of methyldopa or compounds obtained by amidating a hydroxy group of a carboxyl group of methyldopa and then introducing an alkyl group have LO• scavenging ability equivalent to or higher than the LO• scavenging ability of methyldopa and also are superior to methyldopa in terms of achieving a balance between the LOO• scavenging ability and the LO• scavenging ability. Thus, the present invention was completed. The compound of the present invention has, for example, LOO• scavenging ability and LO• scavenging ability and is also superior to methyldopa in terms of a balance between the LOO• scavenging ability and the LO• scavenging ability. In addition to the LOO• scavenging ability and the LO• scavenging ability, the compound of the present invention also may have inhibitory activity against other reactions caused by lipid radicals (L•) and the like in lipid peroxidation. In other words, the compound of the present invention may have, for example, in addition to the inhibitory activity against lipid radical reactions caused by LOO• and/or LO•, inhibitory activity against reactions caused by L• and the like in lipid peroxidation.

As described above, the compound of the present invention has alkoxyl radical scavenging ability. The "alkoxyl radical scavenging ability" in the present invention means the ability to scavenge LO•, for example. The LO• scavenging ability can be evaluated on the basis of its activity value, for example. The activity value of the LO• scavenging ability can be evaluated, for example, using the liposome-$Fe^{2+}$ system disclosed in WO 2019/009355. More specifically, it can be measured in a manner described in Example 1 below. As a specific example, the activity value of the LO• scavenging ability can be measured by performing the following steps (i) to (v):

(i) preparing a buffer solution containing a compound represented by the formula (A) and liposomes;
(ii) adding a divalent iron ion source material;
(iii) adding a test compound;
(iv) measuring a fluorescence; and
(v) determining the activity value of the test compound as per the following equation using the result of the fluorescence measurement.

$$\text{Activity value of LO• scavenging ability} = 1 - (AUC_{Sample}/AUC_{Control})$$

$AUC_{Sample}$: the area under the curve calculated from the fluorescence intensity measured in the presence of $Fe^{2+}$ and the test compound (n = 1)
AUCcontroi: the area under the curve calculated from the fluorescence intensity measured in the presence of $Fe^{2+}$ and in the absence of the test compound

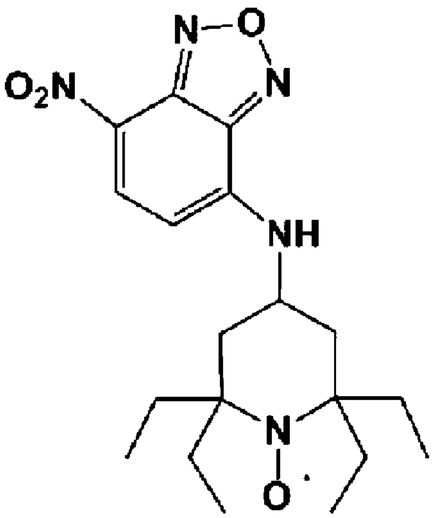

···(A)

In step (i), the concentration of the compound represented by the formula (A) is, for example, 1 to 20 μmol/l and typically 5 μmol/l. The liposomes are prepared from, for example, egg-yolk phosphatidylcholine (Egg PC) and dihexadecyl hydrogen phosphate (DCP). The concentration of the egg-yolk phosphatidylcholine is, for example, 5 to 10 mg/ml and typically 2.5 mg/l. The concentration of the dihexadecyl hydrogen phosphate is, for example, 0.01 to 1 mg/ml and typically 0.1 mg/ml.

In step (ii), the concentration of the divalent iron ion source material is, for example, 0.5 to 5 mmol/l and typically 1 mmol/l. The divalent iron ion source material is, for example, iron(II) sulfate.

In step (iii), the concentration of the test compound is 5 to 100 μmol/l. A specific example of the concentration is 10 μmol/l.

In step (iv), the wavelength of excitation light at the time of measuring the fluorescence can be set to, for example, 470 nm; the fluorescence wavelength can be set to, for example, 530 nm; and the fluorescence intensity at 530 nm can be measured. The period for measuring the fluorescence is, for example, from 0 to 240 minutes at intervals of 0.1 to 5 minutes and typically 180 minutes at intervals of 3 minutes, with step (iii) being the start time, for example.

In the present invention, when the activity value of the LO• scavenging ability of a compound is, for example, greater than that of edaravone, the compound can be regarded as having excellent LO• scavenging ability. The activity value of the LO• scavenging ability is, for example, 0.3 or more and preferably 0.4 or more.

As described above, the compound of the present invention has peroxyl radical scavenging ability. "Peroxyl radical scavenging ability" in the present invention means the ability to scavenge LOO•, for example. The LOO• scavenging ability can be evaluated on the basis of its activity value, for example. The activity value of the LOO• scavenging ability can be evaluated using, for example, the liposome-AAPH (2,2'-azobis(2-amidinopropane)dihydrochloride) system disclosed in WO 2019/009355. More specifically, it can be measured in a manner described in Example 1 below. As a specific example, the activity value of the LOO• scavenging ability can be measured by performing the following steps (vi) to (x):

(vi) preparing a buffer solution containing a compound represented by the formula (A) and liposomes;
(vii) adding 2,2'-azobis(2-amidinopropane)dihydrochloride;
(viii) adding a test compound;
(iv) measuring a fluorescence; and
(x) determining the activity value of the test compound as per the following equation using the result of the fluorescence measurement.

$$\text{Activity value of LOO• scavenging ability} = 1 - (Flu_{Sample} - Flu_{Background})/(Flu_{Control} - Flu_{Background})$$

$Flu_{Sample}$: the fluorescence intensity measured in the presence of AAPH and the test compound (n = 1)
$Flu_{Background}$: the fluorescence intensity measured in the absence of AAPH
Flucontroi: the fluorescence intensity measured in the presence of AAPH and in the absence of the test compound

In step (vi), the concentration of the compound represented by the formula (A) is, for example, 1 to 20 μmol/l and typically 5 μmol/l. The liposomes are prepared from, for example, egg-yolk phosphatidylcholine and dihexadecyl hydrogen phosphate. The concentration of the egg-yolk phosphatidylcholine is, for example, 5 to 10 mg/ml and typically 2.5 mg/ml. The concentration of the dihexadecyl hydrogen phosphate is, for example, 0.01 to 1 mg/ml and typically 0.1 mg/ml.

In step (vii), the concentration of AAPH is, for example, 5 to 50 mmol/l and typically 20 mmol/l.

In step (viii), the concentration of the test compound is 5 to 100 μmol/l. A specific example of the concentration is 10 μmol/l.

In step (ix), the wavelength of excitation light at the time of measuring the fluorescence can be set to, for example, 470 nm; the fluorescence wavelength can be set to, for example, 530 nm; and the fluorescence intensity at 530 nm can be measured. The measurement of the fluorescence is performed, for example, after 1 to 120 minutes has elapsed and typically after 30 to 40 minutes has elapsed since step (viii).

In the present invention, when the activity value of the LOO• scavenging ability of a compound is, for example, greater than that of edaravone, the compound can be regarded as having excellent LOO• scavenging ability. The activity value of the LOO• scavenging ability is, for example, 0.3 or more and preferably 0.4 or more.

In the present invention, the balance between the LO• scavenging ability and the LOO• scavenging ability can be expressed, for example, as the ratio (R/L) of the activity value (R) of the LO• scavenging ability to the activity value (L) of the LOO• scavenging ability. In the present invention, a good balance between the LO• scavenging ability and the LOO• scavenging ability means that, for example, the R/L of a test compound is in the range from 0.5 to 2, from 0.6 to 1.67, from 0.7 to 1.4, or from 0.8 to 1.25. By setting the R/L to fall within such a range, the compound of the present invention can favorably capture both LO• and LOO•, for example. Note here that the R/L of methyldopa is about 0.26.

Each substituent in the compound represented by the formula (1) will be described below with reference to specific examples. In the description regarding each substituent, reference can be made to specific examples given in descriptions regarding other substituents, unless otherwise stated. Unless otherwise stated below, the description regarding the compound represented by the formula (1) also applies to salts of the compound represented by the formula (1), for example.

The compound of the present invention has an asymmetric carbon atom. Thus, the compound of the present invention may be present in the form of, for example, a racemic body, an R- or S-enantiomer thereof, or a mixture of R and S at any ratio. The compound of the present invention may have two or more asymmetric centers. In this case, the compound of the present invention may include diastereomers and mixtures thereof. When the compound of the present invention has a double bond in its molecule, the compound of the present invention may include, for example, those in the form of geometric isomers such as cis isomers and trans isomers. The compound of the present invention also may be present in the form of, for example, a D-isomer, an L-isomer, or a mixture thereof, and preferably in the form of an L-isomer. When the compound of the present invention is in the form of an L-isomer, the compound of the present invention is expected to be efficiently taken up into cells through an amino acid transporter, for example.

$R^1$ and $R^2$ are each a hydrogen atom, an alkyl group, or an acyl group. The alkyl group is, for example, a straight-chain, branched, or cyclic saturated or unsaturated alkyl group having 1 to 6 carbon atoms. Specifically, the alkyl group may be, for example, a methyl group, an ethyl group, an n-propyl group, an iso(i)-propyl group, an n-butyl group, an i-butyl group, a tert(t)-butyl group, a sec(s)-butyl group, an n-pentyl group, an i-pentyl group, a t-pentyl group, an n-hexyl group, an i-hexyl group, a t-hexyl group, an n-heptyl group, an i-heptyl group, a t-heptyl group, an n-octyl group, an i-octyl group, a t-octyl group, an n-nonyl group, an i-nonyl group, a t-nonyl group, an n-decyl group, an i-decyl group, a t-decyl group, an n-undecyl group, an i-undecyl group, an n-dodecyl group, an i-dodecyl group, an n-tridecyl group, an i-tridecyl group, an n-tetradecyl group, an i-tetradecyl group, an n-pentadecyl group, an i-pentadecyl group, an n-hexadecyl group, an i-hexadecyl group, an n-heptadecyl group, an i-heptadecyl group, an n-octadecyl group, an i-octadecyl group, an n-nonadecyl group, or an i-nonadecyl group. Preferably the alkyl group is, for example, a straight-chain saturated alkyl group having 1 to 5 carbon atoms.

The acyl group (alkanoyl group) may be an acyl group having 1 to 6 carbon atoms. The acyl group may be, for example, a formyl group, an acetyl group, a propynyl group, a butyryl group, an iso-butyryl group (iso-valeryl group), a sec-butyryl group, a tert-butyryl group (pivaloyl group), a valeryl group, or a hexanyl group (caproyl group).

$R^1$ and $R^2$ each may be a protecting group, for example. The protecting group may be, for example, TBS (tert-butyldimethylsilyl group), an Fmoc group (fluorenylmethyloxycarbonyl chloride group), a Boc group (tert-butoxycarbonyl group), or a Cbz group (benzyloxycarbonyl group).

In the compound represented by the formula (1), hydrogen in the amino group may be substituted with a protecting group.

In a certain aspect, the compound represented by the formula (1) is represented by the following formula (2), for example.

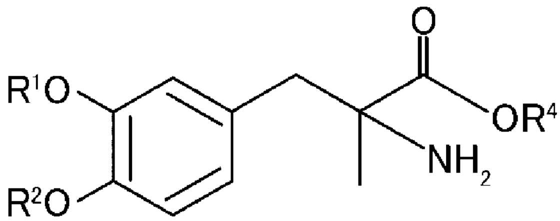

...(2)

In the formula (2),

$R^1$ and $R^2$ may be the same or different and are each independently a hydrogen atom or an alkyl group, and
$R^4$ is a sec-butyl group, a tert-butyl group, or an iso-butyl group.

As a specific example, the compound represented by the formula (1) is preferably a compound represented by the following formula (3). The compound represented by the following formula (3) is, for example, superior in the LO• scavenging ability and the LOO• scavenging ability and also has a good balance between the LO• scavenging ability and the LOO• scavenging ability. The compound represented by the following formula (3) also can be referred to as, for example, s-butyl (2S)-2-amino-3-(3,4-dihydroxyphenyl)-2-methylpropanoate. The compound represented by the following formula (3) may form a hydrochloride.

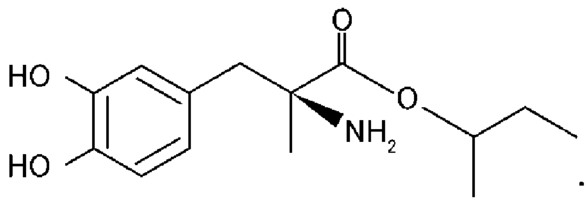

...(3)

As a specific example, the compound represented by the formula (1) is preferably a compound represented by the following formula (4). The compound represented by the following formula (4) is, for example, superior in the LO• scavenging ability and the LOO• scavenging ability and also has a good balance between the LO• scavenging ability and the LOO• scavenging ability. The compound represented by the following formula (4) also can be referred to as, for example, t-butyl (S)-2-amino-3-(3,4-dihydroxyphenyl)-2-methylpropanoate. The compound represented by the following formula (4) may form a hydrochloride.

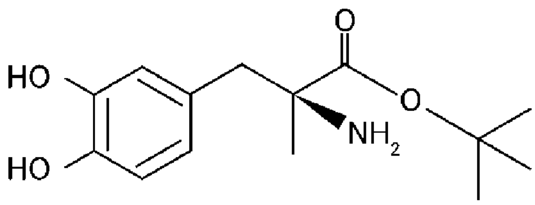

...(4)

As a specific example, the compound represented by the formula (1) is preferably a compound represented by the following formula (5). The compound represented by the following formula (5) is, for example, superior in the LO• scavenging ability and the LOO• scavenging ability and also has a good balance between the LO• scavenging ability and the LOO• scavenging ability. The compound represented by the following formula (5) also can be referred to as, for example, isobutyl (S)-2-amino-3-(3,4-dihydroxyphenyl)-2-methylpropanoate. The compound represented by the following formula (5) may form a hydrochloride.

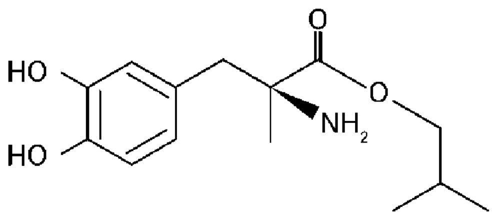

...(5)

In a certain aspect, the compound represented by the formula (1) is represented by the following formula (6), for example.

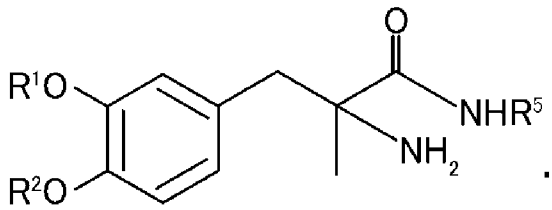

...(6)

In the formula (6),

$R^1$ and $R^2$ may be the same or different and are each independently a hydrogen atom or an alkyl group, and
$R^5$ is a sec-butyl group, a tert-butyl group, or an iso-butyl group.

The compound represented by the formula (1) may be an isomer, for example. The isomer may be, for example, a tautomer or a stereoisomer. The tautomer and the stereoisomer encompass, for example, all theoretically possible tautomers and stereoisomers, respectively. In the present invention, there is no particular limitation on the steric configuration of each substituent. The compound of the present invention may be, for example, a solvate, such as a hydrate, of the compound represented by the formula (1) or a salt thereof. The following description regarding the compound of the present invention also applies to salts of the compound of the present invention and to solvates of the compound of the present invention or a salt thereof.

In the present invention, a salt of the compound of the present invention is not limited to particular salts, and may be, for example, a pharmaceutically acceptable salt. The compound of the present invention forms an acid addition salt or a salt with a base depending on, for example, the type of a substituent. The pharmaceutically acceptable salt is not limited to particular salts, and examples thereof include alkali metal salts such as sodium salts and potassium salts; alkaline earth metal salts such as calcium salts and magnesium salts; ammonium salts; aliphatic amine salts such as trimethylamine salts, triethylamine salts, dicyclohexylamine salts, ethanolamine salts, diethanolamine salts, and triethanolamine salts; aralkylamine salts such as N,N-dibenzylethylenediamine; heterocyclic aromatic amine salts such as pyridine salts, picoline salts, quinoline salts, and isoquinoline salts; quaternary ammonium salts such as tetramethylammonium salts, tetraethylammonium salts, benzyltrimethylammonium salts, benzyltributylammonium salts, methyltrioctylammonium salts, and tetrabutylammonium salts; amino acid salts such as arginine salts, lysine salts, aspartate, and glutamate; inorganic acids such as hydrochloride, sulfate, nitrate, phosphate, carbonate, hydrogencarbonate, and perchlorate; aliphatic or aromatic organic acid salts such as acetate, propionate, succinate, glycolate, lactate, maleate, fumarate, tartrate, malate, citrate, ascorbate, hydroxymaleate, pyruvate, phenylacetate, benzoate, 4-aminobenzoate, anthranilate, 4-hydroxybenzoate, salicylate, 4-aminosalicylate, pamoate, gluconate, and nicotinate; and sulfonates such as methanesulfonate, isethionate, ethanesulphonate, benzenesulfonate, halobenzenesulfonate, p-toluenesulphonate, toluenesulfonate, naphthalenesulfonate, sulfanilate, and cyclohexyl sulfamate.

The compound of the formula (1) and salts thereof can be produced by applying various known synthesis methods utilizing the characteristics derived from their basic structures or the type of the substituent. In the above-described production, for example, depending on the type of the functional group, a protecting group may be introduced before causing a reaction, and thereafter, the protecting group may be removed, if necessary. The compound of the formula (1) or a salt thereof can be produced by, for example, esterifying a carboxyl group of methyldopa or ami dating a hydroxy group of a carboxyl group of methyldopa and then introducing an alkyl group. Alternatively, the compound of the formula (1) or a salt thereof can be produced by, for example, acylating or etherifying a hydroxy group of a catechol group, if necessary.

Pharmaceutical Composition

As described above, the pharmaceutical composition of the present invention contains the compound of the present invention or a salt thereof and a pharmaceutically acceptable carrier. The pharmaceutical composition of the present invention is characterized in that it contains the compound of the present invention or a salt thereof, i.e., the compound represented by the formula (1) or a salt thereof, and there is no particular limitation on other compositions and conditions. As described above, the compound of the present invention or a salt thereof has LO• scavenging ability and LOO• scavenging ability and also is superior to methyldopa in terms of achieving a balance between the LO• scavenging ability and the LOO• scavenging ability. Accordingly, the pharmaceutical composition of the present invention is expected to be applicable to the treatment of diseases caused by radicals, such as diseases for which edaravone is

used for treatment, for example.

The pharmaceutical composition of the present invention may be used in vivo or in vitro, for example.

An administration target of the pharmaceutical composition of the present invention is not limited to particular targets. When the pharmaceutical composition of the present invention is used in vivo, examples of the administration target include humans and non-human animals excluding humans. Examples of the non-human animals include mammals such as mice, rats, rabbits, dogs, sheep, horses, cats, goats, monkeys, and guinea pigs; birds; and fish. When the pharmaceutical composition of the present invention is used in vitro, the administration target may be, for example, a cell, tissue, or an organ. The cell may be, for example, a cell collected from a living organism or a cultured cell. The tissue or organ may be, for example, tissue (biological tissue) or an organ collected from a living organism.

The conditions for use (administration conditions) of the compound of the present invention are not limited to particular conditions, and the administration route, the timing of administration, the dose, and the like can be set as appropriate depending on, for example, the type of the administration target.

The dose of the compound of the present invention is not limited to particular values. When the pharmaceutical composition of the present invention is used in vivo, the dose thereof can be determined as appropriate depending on, for example, the type, symptom, and age of the administration target and the administration method. As a specific example, when the pharmaceutical composition is administered to a human, the total dose of the compound per day is 0.01 to 50 mg/kg, for example. The frequency of administration per day is 1 to 5 times, for example.

The administration route of the pharmaceutical composition of the present invention is not limited to particular forms and may be either oral administration or parenteral administration, for example.

The dosage form of the pharmaceutical composition of the present invention is not limited to particular forms and can be determined as appropriate depending on, for example, the above-described administration route. The dosage form may be, for example, a liquid form or a solid form. When the administration route is oral administration, examples of the dosage form include tablets, pills, capsules, granules, powder medicines, and liquid medicines.

The pharmaceutical composition of the present invention may contain, for example, an additive(s), when necessary. When the pharmaceutical composition of the present invention is used as a medicine or a pharmaceutical composition, the additive preferably includes a pharmaceutically acceptable additive or a pharmaceutically acceptable carrier. The additive is not limited to particular additives, and examples thereof include a base ingredient, an excipient, a coloring agent, a lubricant, a binding agent, a disintegrant, a stabilizing agent, a coating agent, a preservative, and a taste- and odor-masking agent such as fragrance. In the present invention, the blended amount of the additive(s) is not limited to particular values as long as the function of the compound of the present invention is not impaired.

Examples of the excipient include sugar derivatives such as lactose, lactose hydrate, sucrose, glucose, mannitol, and sorbitol; starch derivatives such as corn starch, potato starch, $\alpha$-starch, and dextrin; cellulose derivatives such as crystalline cellulose; gum arabic; dextran; organic excipients such as pullulan; silicate derivatives such as light anhydrous silicic acid, synthetic aluminum silicate, calcium silicate, and magnesium aluminometasilicate; phosphates such as calcium hydrogen phosphate; carbonates such as calcium carbonate; and inorganic excipients including sulphates such as calcium sulfate. Examples of the coloring agent include yellow ferric oxide. Examples of the lubricant include metal stearates such as stearic acid, calcium stearate, and magnesium stearate; talc; polyethylene glycol; silica; and hydrogenated vegetable oils. Examples of the flavoring agent include fragrances such as cocoa powders, menthol, aromatic powders, mentha oils, borneol, and powdered cinnamon bark, sweeteners, and acidulants. Examples of the binding agent include hydroxypropyl cellulose, hydroxypropyl methylcellulose, polyvinyl pyrrolidone, and macrogol. Examples of the disintegrant include cellulose derivatives such as carboxymethyl cellulose and carboxy methylcellulose calcium; chemically modified starches such as carboxymethyl starch, sodium carboxymethyl starch, cross-linked polyvinylpyrrolidone, and sodium starch glycolate; and chemically modified celluloses. Examples of the stabilizing agent include esters of p-hydroxybenzoic acid such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. Examples of the coating agent include hypromellose, macrogol such as macrogol 6000, talc, and titanium oxide.

## Antioxidant

In another aspect, the present invention provides an antioxidant that has a good balance between the LOO• scavenging ability and the LO• scavenging ability. As described above, the antioxidant of the present invention contains a compound represented by the following formula (1A) or a salt thereof.

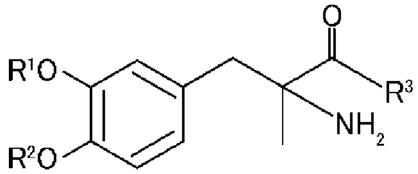

···(1A)

In the formula (1A),

$R^1$ and $R^2$ may be the same or different and are each independently a hydrogen atom, an alkyl group, or an acyl group,
$R^3$ is $-OR^4$ or $-NHR^5$,
$R^4$ is an alkyl group, and
$R^5$ is a hydrogen atom or an alkyl group.

The antioxidant of the present invention is characterized in that it contains the compound represented by the formula (1A) or a salt thereof, and there is no particular limitation on other compositions and conditions. Since the antioxidant of the present invention contains the compound represented by the formula (1A) or a salt thereof, the antioxidant can scavenge radicals. The compound or a salt thereof in the antioxidant of the present invention is superior to methyldopa in terms of a balance between the LOO• scavenging ability and the LO• scavenging ability. Accordingly, the antioxidant of the present invention is presumed to function as an excellent antioxidant in living organisms. In the following, the description regarding the compound in the antioxidant of the present invention also applies to salts of the compound of the present invention and to solvates of the compound of the present invention or a salt thereof, unless otherwise stated. As to the antioxidant of the present invention, reference can be made to the above descriptions regarding the compound or salt and pharmaceutical composition of the present invention.

The "antioxidant" in the present invention means an agent that captures radicals, for example. Examples of the radicals include radical species such as hydroxy radicals (•OH), alkoxy radicals (alkoxyl radicals, LO•), peroxy radicals (peroxyl radicals, LOO•), hydroperoxy radicals (HOO•), nitrogen monoxide (NO•), nitrogen dioxide ($NO_2$•), superoxide anions ($O_2$•), and lipid radicals (L•); and non-radical species such as singlet oxygen ($^1O_2$), ozone ($O_3$), and hydrogen peroxide ($H_2O_2$). The antioxidant of the present invention may capture one of the above-described radicals or two or more of the above-described radicals, for example. In the present invention, the "antioxidant" need only capture at least one of these radicals. Capturing of radicals as described above also can be referred to as, for example, scavenging of radicals. The antioxidant of the present invention captures radicals by donating hydrogen atoms to reactive oxygen species, thereby converting the radicals to other more stable molecules (e.g., water). The antioxidant of the present invention also can be referred to as, for example, a radical scavenger. The antioxidant of the present invention also can prevent, inhibit, or suppress radical-induced oxidation of other molecules that are in the presence of the antioxidant, for example. Accordingly, the antioxidant of the present invention also can be referred to as, for example, an oxidation preventing agent, an oxidation inhibitor, or an oxidation suppressor. The antioxidant of the present invention has the ability to scavenge peroxyl radicals (LOO•) and alkoxyl radicals (LO•) involved in lipid peroxidation. Accordingly, the antioxidant of the present invention also can be referred to as, for example, an agent for preventing, inhibiting, and/or suppressing lipid peroxidation or an agent for preventing, inhibiting, and/or suppressing generation of lipid peroxides. In addition to the LOO• scavenging ability and LO• scavenging ability, the antioxidant of the present invention also may have inhibitory activity against other reactions in lipid peroxidation caused by lipid radicals (L•) and the like. In other words, the antioxidant of the present invention may have, for example, in addition to inhibitory activity against lipid radical reactions caused by LOO• and/or LO•, inhibitory activity against reactions caused by L• and the like in lipid peroxidation.

The radical capturing ability can be evaluated, for example, on the bases of the activity value of the LO• scavenging ability or the activity value of the LOO• scavenging ability, which are as described above. Specifically, a test compound can be regarded as having the ability to capture radicals when the activity value of the LO• scavenging ability thereof exceeds a predetermined value or the activity value of the LOO• scavenging ability thereof exceeds a predetermined value. Specifically, a test compound can be regarded as having LO• scavenging ability when the activity value of the LO• scavenging ability thereof is, for example, 0.25 or more. Also, a test compound can be regarded as having LOO• scavenging ability when the activity value of the LOO• scavenging ability thereof is, for example, 0.25 or more.

As described above, the antioxidant of the present invention has alkoxyl radical scavenging ability. The "alkoxyl radical scavenging ability" in the present invention means the ability to scavenge LO•, for example. The LO• scavenging ability can be evaluated, for example, on the bases of the activity value, as described above.

In the antioxidant of the present invention, the compound can be regarded as having excellent LO• scavenging ability when, for example, the activity value of the LO• scavenging ability is greater than that of edaravone. The activity value of the LO• scavenging ability is, for example, 0.3 or more and preferably 0.4 or more.

In the antioxidant of the present invention, the compound represented by the formula (1A) has peroxyl radical

scavenging ability, as described above. The "peroxyl radical scavenging ability" in the present invention means the ability to scavenge LOO•, for example. The LOO• scavenging ability can be evaluated, for example, on the bases of the activity value, as described above.

The compound represented by the formula (1A) in the antioxidant of the present invention can be regarded as having excellent LOO• scavenging ability when, for example, the activity value of the LOO• scavenging ability is greater than that of edaravone. The activity value of the LOO• scavenging ability is, for example, 0.3 or more and preferably 0.4 or more.

In the antioxidant of the present invention, the balance between the LO• scavenging ability and the LOO• scavenging ability can be expressed, for example, as the ratio (R/L) of the activity value (R) of the LO• scavenging ability to the activity value (L) of the LOO• scavenging ability. In the antioxidant of the present invention, a good balance between the LO• scavenging ability and the LOO• scavenging ability means that, for example, the R/L of a test compound is in the range from 0.5 to 2, from 0.6 to 1.67, from 0.7 to 1.4, or from 0.8 to 1.25. By setting the R/L to fall within such a range, the antioxidant of the present invention can favorably capture both LO• and LOO•, for example. Note here that the R/L of methyldopa is about 0.26.

Each substituent in the compound represented by the formula (1A) will be described below with specific examples. In the description regarding each substituent, reference can be made to specific examples given in the descriptions regarding other substituents, unless otherwise stated. Unless otherwise stated below, the description regarding the compound represented by the formula (1A) also applies to salts of the compound represented by the formula (1A), for example.

The compound represented by the formula (1A) has an asymmetric carbon atom. Thus, the compound represented by the formula (1A) may be present in the form of, for example, a racemic body, an R- or S-enantiomer thereof, or a mixture of R and S at any ratio. The compound represented by the formula (1A) may have two or more asymmetric centers. In this case, the compound represented by the formula (1A) may include diastereomers and mixtures thereof. When the compound represented by the formula (1A) has a double bond in its molecule, the compound represented by the formula (1A) may include, for example, those in the form of geometric isomers such as cis isomers and trans isomers. The compound represented by the formula (1A) also may be present in the form of, for example, a D-isomer, an L-isomer, or a mixture thereof, and preferably in the form of an L-isomer. When the compound represented by the formula (1A) is in the form of an L-isomer, the compound represented by the formula (1A) is expected to be efficiently taken up into cells through an amino acid transporter, for example.

$R^1$ and $R^2$ are each a hydrogen atom, an alkyl group, or an acyl group. The alkyl group is, for example, a straight-chain, branched, or cyclic saturated or unsaturated alkyl group having 1 to 6 carbon atoms. Specifically, the alkyl group may be, for example, a methyl group, an ethyl group, an n-propyl group, an iso(i)-propyl group, an n-butyl group, an i-butyl group, a tert(t)-butyl group, a sec(s)-butyl group, an n-pentyl group, an i-pentyl group, a t-pentyl group, an n-hexyl group, an i-hexyl group, a t-hexyl group, an n-heptyl group, an i-heptyl group, a t-heptyl group, an n-octyl group, an i-octyl group, a t-octyl group, an n-nonyl group, an i-nonyl group, a t-nonyl group, an n-decyl group, an i-decyl group, a t-decyl group, an n-undecyl group, an i-undecyl group, an n-dodecyl group, an i-dodecyl group, an n-tridecyl group, an i-tridecyl group, an n-tetradecyl group, an i-tetradecyl group, an n-pentadecyl group, an i-pentadecyl group, an n-hexadecyl group, an i-hexadecyl group, an n-heptadecyl group, an i-heptadecyl group, an n-octadecyl group, an i-octadecyl group, an n-nonadecyl group, or an i-nonadecyl group. Preferably, the alkyl group is, for example, a straight-chain saturated alkyl group having 1 to 5 carbon atoms.

The acyl group (alkanoyl group) may be an acyl group having 1 to 6 carbon atoms. The acyl group may be, for example, a formyl group, an acetyl group, a propynyl group, a butyryl group, an iso-butyryl group (iso-valeryl group), a sec-butyryl group, a tert-butyryl group (pivaloyl group), a valeryl group, or a hexanyl group (caproyl group).

$R^1$ and $R^2$ each may be a protecting group, for example. The protecting group may be, for example, TBS (tert-butyldimethylsilyl group), an Fmoc group (fluorenylmethyloxycarbonyl chloride group), a Boc group (tert-butoxycarbonyl group), or a Cbz group (benzyloxycarbonyl group).

$R^4$ is an alkyl group. The alkyl group preferably is an ethyl group, a propyl group, a sec-butyl group, a tert-butyl group, or an iso-butyl group, and more preferably is a sec-butyl group or a tert-butyl group.

$R^5$ is a hydrogen atom or an alkyl group. The alkyl group preferably is an ethyl group, a propyl group, a sec-butyl group, a tert-butyl group, or an iso-butyl group, and more preferably is a sec-butyl group or a tert-butyl group.

In the compound represented by the formula (1A), hydrogen in the amino group may be substituted with the above-described protecting group.

In a certain aspect, the compound represented by the formula (1A) is represented by the following formula (2), for example.

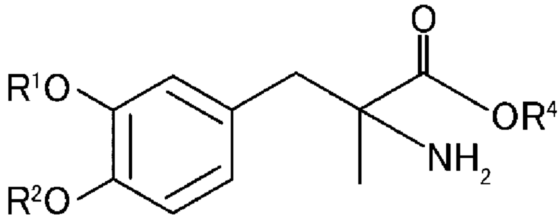

...(2)

In the formula (2),

$R^1$ and $R^2$ may be the same or different and are each independently a hydrogen atom or an alkyl group, and
$R^4$ is an alkyl group.

As a specific example, the compound represented by the formula (1A) is preferably a compound represented by the following formula (3). The compound represented by the following formula (3) is, for example, superior in the LO• scavenging ability and the LOO• scavenging ability and also achieves a good balance between the LO• scavenging ability and the LOO• scavenging ability. The compound represented by the following formula (3) also can be referred to as, for example, s-butyl (2S)-2-amino-3-(3,4-dihydroxyphenyl)-2-methylpropanoate. The compound represented by the following formula (3) may form a hydrochloride.

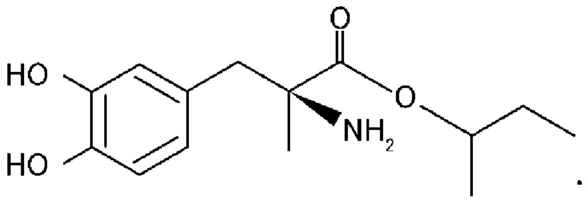

...(3)

As a specific example, the compound represented by the formula (1A) is preferably a compound represented by the following formula (4). The compound represented by the following formula (4) is, for example, superior in the LO• scavenging ability and the LOO• scavenging ability and also achieves a good balance between the LO• scavenging ability and the LOO• scavenging ability. The compound represented by the following formula (4) also can be referred to as, for example, t-butyl (S)-2-amino-3-(3,4-dihydroxyphenyl)-2-methylpropanoate. The compound represented by the following formula (4) may form a hydrochloride.

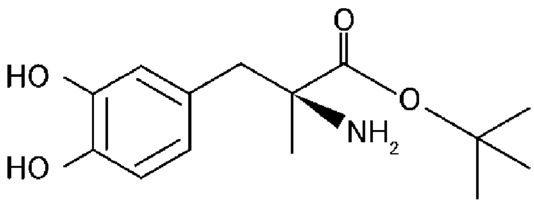

...(4)

As a specific example, the compound represented by the formula (1A) is preferably a compound represented by the following formula (5). The compound represented by the following formula (5) is, for example, superior in the LO• scavenging ability and the LOO• scavenging ability and also has a good balance between the LO• scavenging ability and the LOO• scavenging ability. The compound represented by the following formula (5) also can be referred to as, for example, isobutyl (S)-2-amino-3-(3,4-dihydroxyphenyl)-2-methylpropanoate. The compound represented by the following formula (5) may form a hydrochloride.

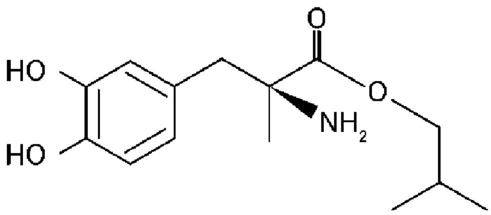

...(5)

As a specific example, the compound represented by the formula (1A) is preferably a compound represented by the following formula (7). The compound represented by the following formula (7) is, for example, superior in the LO• scavenging ability and the LOO• scavenging ability and also has a good balance between the LO• scavenging ability and the LOO• scavenging ability. The compound represented by the following formula (7) also can be referred to as, for example, propyl (S)-2-amino-3-(3,4-dihydroxyphenyl)-2-methylpropanoate. The compound represented by the following formula (7) may form a hydrochloride.

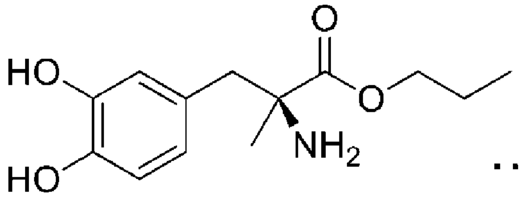

···(7)

As a specific example, the compound represented by the formula (1A) is preferably a compound represented by the following formula (8). The compound represented by the following formula (8) is, for example, superior in the LO• scavenging ability and the LOO• scavenging ability and also has a good balance between the LO• scavenging ability and the LOO• scavenging ability. The compound represented by the following formula (8) also can be referred to as, for example, isopropyl (S)-2-amino-3-(3,4-dihydroxyphenyl)-2-methylpropanoate. The compound represented by the following formula (8) may form a hydrochloride.

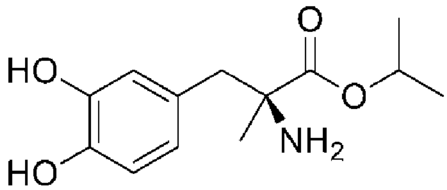

···(8)

As a specific example, the compound represented by the formula (1A) is preferably a compound represented by the following formula (9). The compound represented by the following formula (9) is, for example, superior in the LO• scavenging ability and the LOO• scavenging ability and also has a good balance between the LO• scavenging ability and the LOO• scavenging ability. The compound represented by the following formula (9) also can be referred to as, for example, butyl (S)-2-amino-3-(3,4-dihydroxyphenyl)-2-methylpropanoate. The compound represented by the following formula (9) may form a hydrochloride.

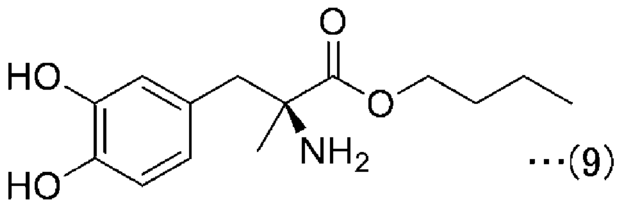

···(9)

As a specific example, the compound represented by the formula (1A) is preferably a compound represented by the following formula (10). The compound represented by the following formula (10) is, for example, superior in the LO• scavenging ability and the LOO• scavenging ability and also has a good balance between the LO• scavenging ability and the LOO• scavenging ability. The compound represented by the following formula (10) also can be referred to as, for example, ethyl (S)-2-amino-3-(3,4-dihydroxyphenyl)-2-methylpropanoate. The compound represented by the following formula (10) may form a hydrochloride.

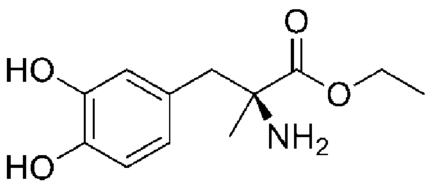

···(10)

As a specific example, the compound represented by the formula (1A) is a compound represented by the following formula (11). The compound represented by the following formula (11) also can be referred to as, for example, methyl (S)-2-amino-3-(3,4-dihydroxyphenyl)-2-methylpropanoate. The compound represented by the following formula (11) may form a hydrochloride.

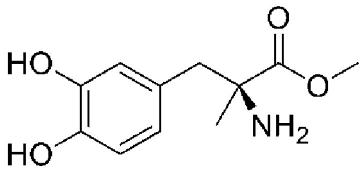

...(11)

In a certain aspect, the compound represented by the formula (1A) is represented by the following formula (6), for example.

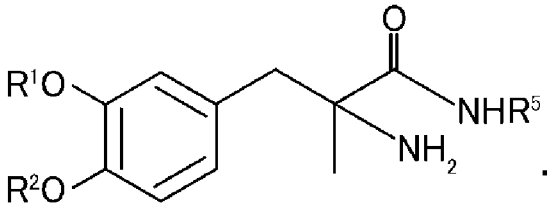

...(6)

In the formula (6),

$R^1$ and $R^2$ may be the same or different and are each independently a hydrogen atom or an alkyl group, and
$R^5$ is a hydrogen atom or an alkyl group.

As a specific example, the compound represented by the formula (1A) is a compound represented by the following formula (12). The compound represented by the following formula (12) also can be referred to as, for example, (S)-2-amino-3-(3,4-dihydroxyphenyl)-2-methylpropanamide. The compound represented by the following formula (12) may form a hydrochloride.

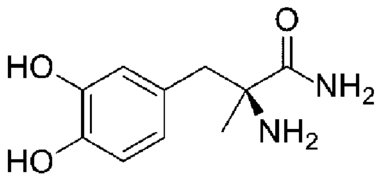

...(12)

The compound represented by the formula (1A) may be an isomer, for example. The isomer may be, for example, a tautomer or a stereoisomer. The tautomer and the stereoisomer encompass, for example, all theoretically possible tautomers and stereoisomers, respectively. In the present invention, there is no particular limitation on the steric configuration of each substituent. The antioxidant of the present invention may contain, for example, a solvate, such as a hydrate, of the compound represented by the formula (1A) or a salt thereof. The following description regarding the compound represented by the formula (1A) also applies to salts of the compound of the present invention and to solvates of the compound of the present invention or a salt thereof.

In the antioxidant of the present invention, a salt of the compound represented by the formula (1A) is not limited to particular salts, and may be, for example, a pharmaceutically acceptable salt. The compound represented by the formula (1A) forms an acid addition salt or a salt with a base depending on, for example, the type of a substituent. The pharmaceutically acceptable salt is not limited to particular salts, and examples thereof include alkali metal salts such as sodium salts and potassium salts; alkaline earth-metal salts such as calcium salts and magnesium salts; ammonium salts; aliphatic amine salts such as trimethylamine salts, triethylamine salts, dicyclohexylamine salts, ethanolamine salts, diethanolamine salts, and triethanolamine salts; aralkylamine salts such as N,N-dibenzylethylenediamine; heterocyclic aromatic amine salts such as pyridine salts, picoline salts, quinoline salts, and isoquinoline salts; quaternary ammonium salts such as tetramethylammonium salts, tetraethylammonium salts, benzyltrimethylammonium salts, benzyltributylammonium salts, methyltrioctylammonium salts, and tetrabutylammonium salts; amino acid salts such as arginine salts, lysine salts, aspartate, and glutamate; inorganic acids such as hydrochloride, sulfate, nitrate, phosphate, carbonate,

hydrogencarbonate, and perchlorate; aliphatic or aromatic organic acid salts such as acetate, propionate, succinate, glycolate, lactate, maleate, fumarate, tartrate, malate, citrate, ascorbate, hydroxymaleate, pyruvate, phenylacetate, benzoate, 4-aminobenzoate, anthranilate, 4-hydroxybenzoate, salicylate, 4-aminosalicylate, pamoate, gluconate, and nicotinate; and sulfonates such as methanesulfonate, isethionate, ethanesulphonate, benzenesulfonate, halobenzenesulfonate, p-toluenesulphonate, toluenesulfonate, naphthalenesulfonate, sulfanilate, and cyclohexyl sulfamate.

The compound of the formula (1A) and salts thereof can be produced by applying various known synthesis methods utilizing the characteristics derived from their basic structures or the type of the substituent. In the above-described production, for example, depending on the type of the functional group, a protecting group may be introduced before causing a reaction, and thereafter, the protecting group may be removed, if necessary. The compound of the formula (1A) or a salt thereof can be produced by, for example, esterifying a carboxyl group of methyldopa or amidating a hydroxy group of a carboxyl group of methyldopa and then introducing an alkyl group. Alternatively, the compound of the formula (1A) or a salt thereof can be produced by, for example, acylating or etherifying a hydroxy group of a catechol group, if necessary.

The administration conditions of the antioxidant of the present invention are not limited to particular conditions, and the administration route, the timing of administration, the dose, and the like can be set as appropriate depending on, for example, the type of an administration target. As to the administration target and the administration conditions of the antioxidant of the present invention, reference can be made to the above descriptions regarding the administration target and the administration conditions of the compound of the present invention.

Cell Protectant

In still another aspect, the present invention provides a compound that has a good balance between the LOO• scavenging ability and the LO• scavenging ability and thus exhibits cell-protection activity. The cell protectant (hereinafter also referred to simply as "protectant") of the present invention contains the compound of the present invention or a salt thereof or the antioxidant of the present invention, as described above. The protectant of the present invention is characterized in that it contains the compound of the present invention or a salt thereof or the antioxidant of the present invention, i.e., a compound represented by the formula (1) or (1A) or a salt thereof, and there is no particular limitation on other compositions and conditions. Since the protectant of the present invention contains the compound represented by the formula (1) or (1A) or a salt thereof, the protectant can scavenge radicals. Accordingly, the cell protectant of the present invention is presumed to be able to suppress cell disorder caused by the radicals. As to the protectant of the present invention, reference can be made to the above descriptions regarding the compound or salt, pharmaceutical composition, and antioxidant of the present invention.

The "cell protection" in the present invention need only mean that cell disorder is (significantly) suppressed as compared with that in the absence of the cell protectant of the present invention (unadministered conditions), and cell disorder may have advanced as compared with the state at the start (i.e., the start of administration). In this case, the "cell protection" also can be referred to as, for example, "suppression of cell disorder" or the like. The cell disorder means, for example, hypofunction or dysfunction of cells and can be evaluated based on the metabolism, membrane permeability, and the like of the cells.

For example, cells may be cells such as those collected from a living organism or cultured cells, or they may be a cell sheet composed of cells, a cell cluster (spheroid), tissue, or an organ.

The protectant of the present invention can be suitably used, for example, for protecting cells from cell death caused by radicals and especially from cell death caused by ferroptosis. "Ferroptosis" means cell death caused by oxidized lipids.

The administration conditions of the cell protectant of the present invention are not limited to particular conditions, and the administration route, the timing of administration, the dose, and the like can be set as appropriate depending on, for example, the type of an administration target. As to the administration target and the administration conditions of the cell protectant of the present invention, reference can be made to the above descriptions regarding the administration target and the administration conditions of the compound of the present invention.

Cell Growth-Promoting Agent

In still another aspect, the present invention provides a compound capable of promoting cell growth. The cell growth-promoting agent (hereinafter also referred to simply as "promoting agent") of the present invention contains the compound of the present invention or a salt thereof or the antioxidant of the present invention, as described above. The promoting agent of the present invention is characterized in that it contains the compound of the present invention or a salt thereof or the antioxidant of the present invention, i.e., the compound represented by the formula (1) or (1A) or a salt thereof, and there is no particular limitation on other compositions and conditions. The promoting agent of the present invention can promote cell growth by containing the compound represented by the formula (1) or (1A) or a salt thereof.

As to the protectant of the present invention, reference can be made to the above descriptions regarding the compound or salt, pharmaceutical composition, and antioxidant of the present invention.

The "promotion of cell growth" in the present invention need only mean that cell growth is (significantly) improved as compared with that in the absence of the promoting agent of the present invention (unadministered conditions), and the cell growth may be reduced as compared with the state at the start (i.e., the start of administration). In this case, the "promotion of cell growth" also can be referred to as, for example, "suppression of reduction of cell growth" or the like.

For example, cells may be cells such as those collected from a living organism or cultured cells, or they may be a cell sheet composed of cells, a cell cluster (spheroid), tissue, or an organ. The cells may be neuronal cells, for example.

The administration conditions of the promoting agent of the present invention are not limited to particular conditions, and the administration route, the timing of administration, the dose, and the like can be set as appropriate depending on, for example, the type of administration target. As to the administration target and the administration conditions of the promoting agent of the present invention, reference can be made to the above descriptions regarding the administration target and the administration conditions of the compound of the present invention.

Oxidation Prevention Method

In still another aspect, the present invention provides an oxidation prevention method that uses well-balanced LOO• scavenging ability and LO• scavenging ability. The oxidation prevention method of the present invention uses the antioxidant of the present invention, as described above. The oxidation prevention method of the present invention is characterized in that it uses the antioxidant of the present invention, i.e., a compound represented by the formula (1A) or a salt thereof, and there is no particular limitation on other steps and conditions. Since the oxidation prevention method of the present invention uses the antioxidant of the present invention, radicals can be captured by this method. Accordingly, the oxidation prevention method of the present invention can prevent oxidation of other molecules that are in the presence of the antioxidant, for example. As to the oxidation prevention method of the present invention, reference can be made to the above descriptions regarding the compound or salt, pharmaceutical composition, and antioxidant of the present invention.

The oxidation prevention method of the present invention can suppress or inhibit radical-induced oxidation of other molecules that are in the presence of the antioxidant, for example. Accordingly, the oxidation prevention method of the present invention also can be referred to as, for example, an oxidation suppression method or an oxidation inhibition method. The antioxidant of the present invention has ability to scavenge peroxyl radicals (LOO•) and alkoxyl radicals (LO•) involved in lipid peroxidation. Accordingly, the oxidation prevention method of the present invention also can be referred to as, for example, a method for preventing, inhibiting, and/or suppressing lipid peroxidation or a method for preventing, inhibiting, and/or suppressing generation of lipid peroxides.

The oxidation prevention method of the present invention includes, for example, the step of bringing a target substance and the antioxidant into contact with each other. More specifically, the oxidation prevention method of the present invention includes, for example, the step of bringing an antioxidation target substance and the antioxidant into contact with each other. The oxidation prevention method of the present invention may include, for example, the step of causing a target substance and the antioxidant to be present together (hereinafter, the step of causing the state of being present together also may be referred to as the "co-locating step"), in addition to or instead of the above-described contact step. More specifically, the co-locating step is the step of, for example, causing an antioxidation target substance and the antioxidant to be present together. The state of being "present together" described above means that, for example, the antioxidant is present simultaneously with the antioxidation target substance in the same agent, the same composition, or the same space that is isolated from other components.

The antioxidation target substance is not limited to particular substances and may be any substance.

The oxidation prevention method of the present invention may use the compound of the present invention or a salt thereof instead of or in addition to the antioxidant of the present invention.

In the oxidation prevention method of the present invention, the contact step and the co-locating step may be performed in vitro or in vivo, for example. As to an administration target and administration conditions of the antioxidant of the present invention, reference can be made to the above descriptions regarding the administration target and the administration conditions of the compound of the present invention.

Cell-Protection Method

In still another aspect, the present invention provides a method capable of protecting cells by using well-balanced LOO• scavenging ability and LO• scavenging ability. The cell-protection method (hereinafter also referred to simply as "protection method") of the present invention uses the cell protectant of the present invention, as described above. The protection method of the present invention is characterized in that it uses the cell protectant of the present invention, i.e., the compound represented by the formula (1) or (1A) or a salt thereof, and there is no particular limitation on other

steps and conditions. Since the protection method of the present invention uses the protectant of the present invention, radicals can be captured by this method. Accordingly, the protection method of the present invention is expected to be able to suppress cell disorder caused by the radicals. As to the protection method of the present invention, reference can be made to the above descriptions regarding the compound or salt, antioxidant, pharmaceutical composition, and cell protectant of the present invention.

The protection method of the present invention includes, for example, the step of causing a cell and the cell protectant to be present together. In this co-locating step, the cell and the cell protectant may be brought into contact with each other. In this case, the co-locating step also can be referred to as a contact step, for example.

In the protection method of the present invention, the co-locating step may be performed in vitro or in vivo, for example. As to an administration target and administration conditions of the protectant of the present invention, reference can be made to the above descriptions regarding the administration target and the administration conditions of the antioxidant of the present invention.

Cell Growth-Promotion Method

In still another aspect, the present invention provides a method capable of promoting cell growth. The cell growth-promotion method (hereinafter also referred to simply as "promotion method") of the present invention uses the promoting agent of the present invention, as described above. The promotion method of the present invention is characterized in that it uses the promoting agent of the present invention, i.e., the compound represented by the formula (1) or (1A) or a salt thereof, and there is no particular limitation on other steps and conditions. Since the promotion method of the present invention uses the promoting agent of the present invention, cell growth can be promoted by this method. As to the promotion method of the present invention, reference can be made to the above descriptions regarding the compound or salt, antioxidant, pharmaceutical composition, cell protectant, and promoting agent of the present invention.

The promotion method of the present invention includes, for example, the step of causing a cell and the promoting agent to be present together. In this co-locating step, the cell and the promoting agent may be brought into contact with each other. In this case, the co-locating step also can be referred to as a contact step, for example.

In the promotion method of the present invention, the co-locating step may be performed in vitro or in vivo, for example. As to an administration target and administration conditions of the promoting agent of the present invention, reference can be made to the above descriptions regarding the administration target and the administration conditions of the antioxidant of the present invention.

Use of Compound or Salt Thereof

The present invention relates to use of the compound represented by the formula (1) or (1A) or a salt thereof for use in antioxidation; the compound represented by the formula (1) or (1A) or a salt thereof for use in cell protection, or use thereof; and the compound represented by the formula (1) or (1A) or a salt thereof for use in promotion of cell growth, or use thereof. The present invention also relates to use of the compound represented by the formula (1) or (1A) or a salt thereof for use in production of an antioxidant; use of the compound represented by the formula (1) or (1A) or a salt thereof for use in production of a cell protectant; and use of the compound represented by the formula (1) or (1A) or a salt thereof for use in production of a promoting agent. The compound represented by the formula (1) or (1A) may be the compound represented by the formula (1) used as the compound of the present invention, or the compound represented by the formula (1) or (1A) used in the antioxidant of the present invention. As to the present invention, reference can be made to the above descriptions regarding the compound or salt, pharmaceutical composition, antioxidant, protectant, promoting agent, oxidation prevention method, protection method, and promotion method of the present invention. Examples

Next, examples of the present invention will be described. It is to be noted, however, that the present invention is by no means limited by the following exemplary examples. Commercially available reagents in the examples were used in accordance with their protocols, unless otherwise stated.

Example 1

Compounds represented by the formulae (3) to (5) and (7) to (9) were obtained according to synthesis examples to be described below.

Electrospray ionization mass spectrometry (ESI-MS) was performed in a positive-ion mode using a mass spectrometer (LCMS-2020, Shimadzu Corporation). The analysis conditions were as follows.

Interface:

Interface: ESI
DL Temperature: 250°C
Nebulizing Gas Flow: 1.5 l/min
Heat Block: 250°C
Drying Gas: On (15.00 l/min)

Column:

Column Name: Ascentis Express C18 (SIGMA)
Length: 50 mm
Internal Diameter: 3.0 mm
Description: 2.7 μm

Pumps:

Mode: Binary gradient
Pump A: LC-30AD
Pump B: LC-30AD
Total Flow: 1.5 ml/min
B Conc.: 5.0%

Oven:

Oven Temperature: 40°C

PDA:

PDA Model: SPD-M20A
Lamp: D2
Start Wavelength: 190 nm
End Wavelength: 400 nm

Solvents:

Mobile phase A: water/0.05% trifluoroacetic acid (TFA)
Mobile phase B: acetonitrile/0.05% TFA

Time Program (gradient):

| Time (minutes) | 0 | 2 | 2.7 |
|---|---|---|---|
| Mobile phase A | 95 | 0 | 0 |
| Mobile phase B | 5 | 100 | 100 |

(Synthesis of Compound (1b))

A compound (1b) was synthesized from methyldopa (compound (1a)) according to Scheme 1 below.

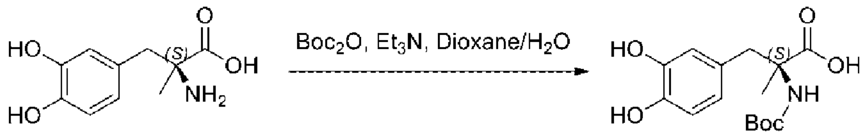


Methyldopa (1a) scheme 1 (1b)

(Synthesis of Compound (1c))

A compound (1c) was synthesized from the compound (1b) according to Scheme 2 below.

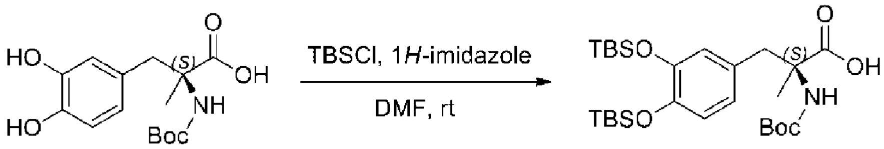


(1b) scheme 2 (1c)

(Synthesis of Compound (3a))

A compound (3a) was synthesized from the compound (1c) according to Scheme 3 below.

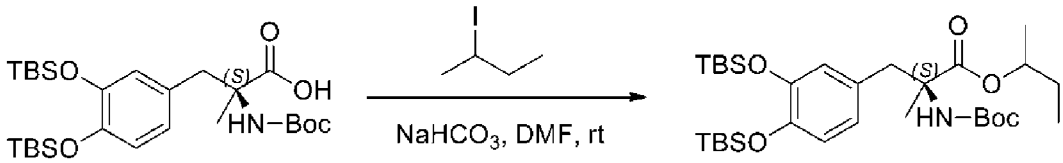


(1c) scheme 3 (3a)

(Synthesis of Compound (3))

A compound (3) was synthesized from the compound (3a) according to Scheme 4 below. The physical properties thereof are shown below. Also, the result of NMR measurement is shown in FIG. 1.

ESI-MS (positive-ion mode) m/z, found = 268.15, calculated for [(M+H)+] = 268.15

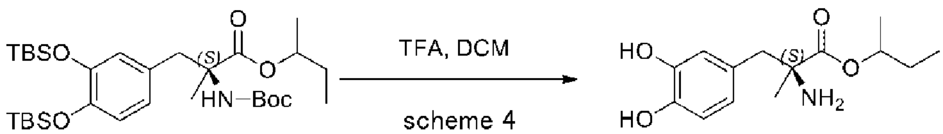


(3a) (3)

(Synthesis of Compound (1d))

A compound (Id) was synthesized from the compound (1c) according to Scheme 5 below.

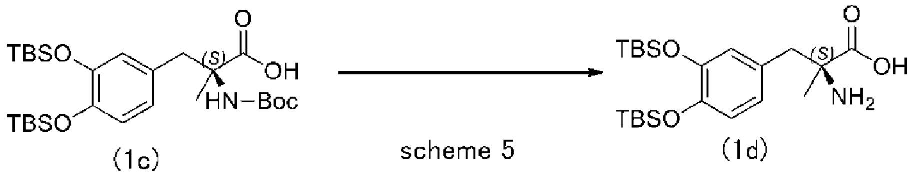

(1c) scheme 5 (1d)

(Synthesis of Compound (4a))

A compound (4a) was synthesized from the compound (Id) according to Scheme 6 below.

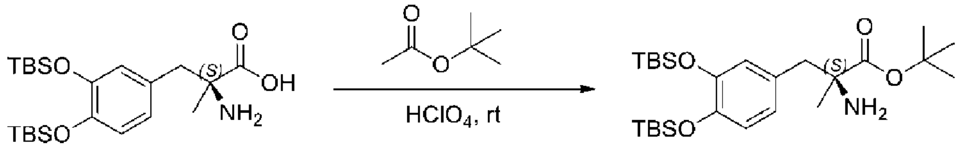


(1d) scheme 6 (4a)

(Synthesis of Compound (4))

A compound (4) was synthesized from the compound (4a) according to Scheme 7 below. The physical properties thereof are shown below. Also, the result of NMR measurement is shown in FIG. 2.

ESI-MS (positive-ion mode) m/z, found = 268.05, calculated for [(M+H)+] = 268.15

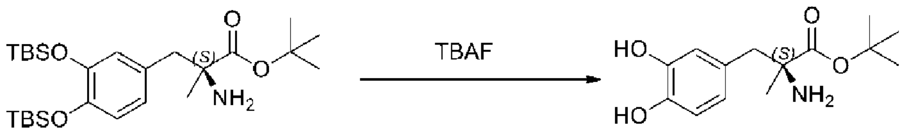


(4a) scheme 7 (4)

(Synthesis of Compound (5))

A compound (5) was synthesized from the compound (1a) according to Scheme 8 below. The physical properties thereof are shown below. Also, the result of NMR measurement is shown in FIG. 3.

ESI-MS (positive-ion mode) m/z, found = 268.10, calculated for [(M+H)+] = 268.15

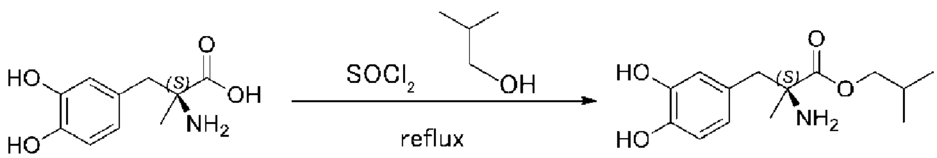


(1a) scheme 8 (5)

(Synthesis of Compound (7))

A compound (7) was synthesized from the compound (1c) according to Scheme 9 below.

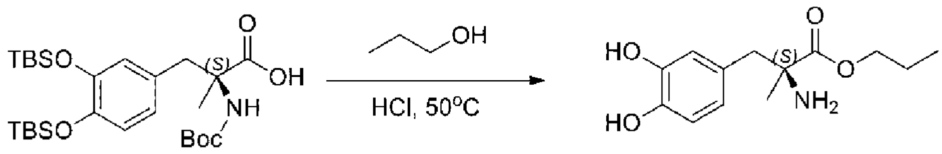


(1c) scheme 9 (7)

(Synthesis of Compound (8a))

A compound (8a) was synthesized from the compound (1c) according to Scheme 10 below.

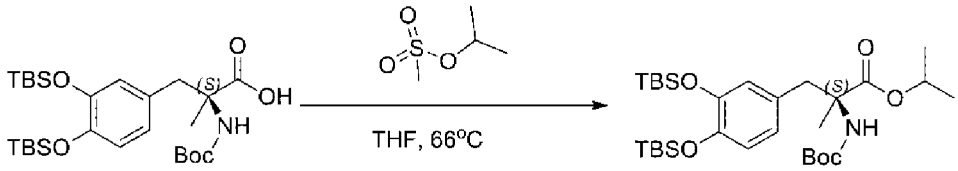


(1c) scheme 10 (8a)

(Synthesis of Compound (8))

A compound (8) was synthesized from the compound (8a) according to Scheme 11 below.

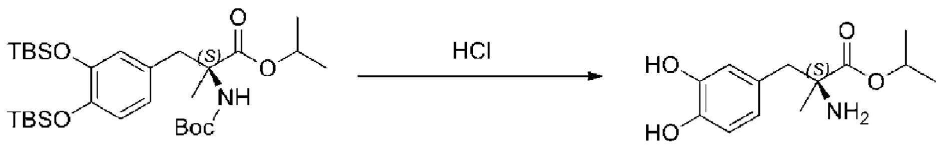


(8a) scheme 11 (8)

(Synthesis of Compound (9a))

A compound (9a) was synthesized from the compound (1c) according to Scheme 12 below.

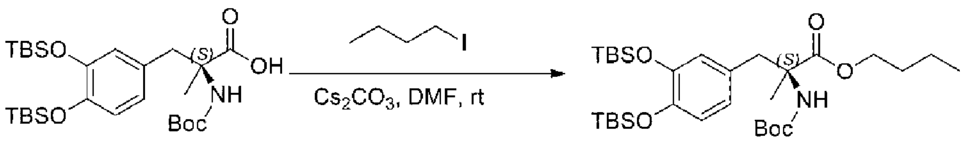


(1c) scheme 12 (9a)

(Synthesis of Compound (9))

A compound (9) was synthesized from the compound (9a) according to Scheme 13 below.

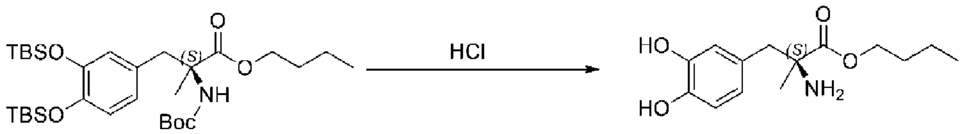


(9a) scheme 13 (9)

Example 2

The present example was performed in order to confirm whether the compound or antioxidant of the present invention has LO• scavenging ability and LOO• scavenging ability and also is superior to methyldopa in terms of a balance between the LO• scavenging ability and the LOO• scavenging ability.

(1) Measurement of Activity Value of LO• Scavenging Ability

The following solutions were prepared: a solution A containing liposomes (10 mg/ml Egg PC, 0.4 mg/ml DCP) and 20 μmol/l NBD-TEEPO compound (compound represented by the formula (A)) in phosphate buffer (10 mmol/l, pH 7.4, 2.0% acetonitrile); and a solution B containing 4.0 mmol/l $FeSO_4$ in distilled water. A 10 μl aliquot of each of the solutions A and B was dispensed using a Multidrop Combi (Thermo Fisher Scientific), and these aliquots were mixed together. The final concentrations of the respective components in the phosphate buffer (10 mmol/l, pH 7.4, 0.5% acetonitrile, 1% dimethyl sulfoxide (DMSO)) were as follows: the liposomes (2.5 mg/ml Egg PC, 0.1 mg/ml DCP), the NBD-TEEPO compound (5.0 μmol/l), a test compound (10 μmol/l), and $FeSO_4$ (1.0 mmol/l). As the test compound, the compounds represented by the formulae (3) to (5) and (7) to (10) were used as compounds of examples, the compound (methyldopa) represented by the formula (1a) was used as a compound of a comparative example, and edaravone and a SCREEN-WELL® FDA-approved drug library (Enzo Life Sciences) were used as reference examples. The test compounds were dissolved in DMSO.

While incubating the thus-obtained mixture at 37°C, the fluorescence intensity was measured over time for 180 minutes at intervals of 3 minutes with the start time set to the start of mixing the components. The fluorescence intensity was measured at an excitation wavelength of 470 nm and a fluorescence wavelength of 530 nm. Then the measured fluorescence intensities were plotted to obtain a curve, and, on the basis of this curve, the activity value of the LO• scavenging ability was calculated as per the above-described equation.

(2) Measurement of Activity Value of LOO• Scavenging Ability

The following solutions were prepared: a solution A containing liposomes (10 mg/ml Egg PC, 0.4 mg/ml DCP) and NBD-TEEPO compound (20 μmol/l) in phosphate buffer (10 mmol/l, pH 7.4, 2.0% acetonitrile); and a solution B containing 80 mmol/l AAPH in phosphate buffer (10 mmol/l, pH 7.4). A 10 μl aliquot of each of the solutions A and B was dispensed using a Multidrop Combi, and these aliquots were mixed together. The final concentrations of the respective components in the phosphate buffer (10 mmol/l, pH 7.4, 0.5% acetonitrile, 1% DMSO) were as follows: the liposomes (2.5 mg/ml Egg PC, 0.1 mg/ml DCP), the NBD-TEEPO compound (5.0 μmol/l), each of the test compounds (10 μmol/l), and AAPH (20 mmol/l).

While incubating the thus-obtained mixture at 37°C, the fluorescence intensity was measured after 40 minutes had elapsed since the mixing of the components. The fluorescence intensity was measured at an excitation wavelength of 470 nm and a fluorescence wavelength of 530 nm. Then, on the basis of the thus-measured fluorescence intensities, the activity value of the LOO• scavenging ability was calculated as per the above-described equation.

(3) Results

The results obtained are shown in FIG. 4 and Table 1 below. FIG. 4 is a graph showing the LO• scavenging ability and the LOO• scavenging ability. In FIG. 4, the horizontal axis indicates the LOO• scavenging ability (AAPH stimulation), and the vertical axis indicates the LO• scavenging ability (Fe stimulation). As can be seen from FIG. 4 and Table 1 below, the compounds of the examples were all superior to edaravone in the LO• scavenging ability and the LOO• scavenging ability. The R/L of methyldopa was 0.26, whereas the R/L values of the compounds of the formulae (3) to (5) and (7) to (10) were 1.04, 1.07, 1.19, 0.88, 0.95, 1.04, and 0.78, respectively, and these compounds all had a good balance between the LO• scavenging ability and the LOO• scavenging ability. Moreover, the compounds of the formulae (3) to (5) were superior to methyldopa in the LO• scavenging ability.

[Table 1]

| | Formula (3): sec-butyl | Formula (4): tert-butyl | Formula (5): iso-butyl | Methyldopa | Edaravone |
|---|---|---|---|---|---|
| Fe stimulation (R) | 0.754 | 0.654 | 0.539 | 0.238 | 0.288 |
| AAPH stimulation (L) | 0.726 | 0.610 | 0.452 | 0.929 | 0.293 |
| R/L | 1.04 | 1.07 | 1.19 | 0.26 | 0.98 |

(continued)

| | Formula (3): sec-butyl | Formula (4): tert-butyl | Formula (5): iso-butyl | Methyldopa | Edaravone |
|---|---|---|---|---|---|
| | Formula (7): propyl | Formula (8): Isopropyl | Formula (9): n-butyl | Formula (10): iso-butyl | |
| Fe stimulation (R) | 0.454 | 0.327 | 0.313 | 0.517 | |
| AAPH stimulation (L) | 0.517 | 0.346 | 0.301 | 0.663 | |
| R/L | 0.88 | 0.95 | 1.04 | 0.78 | |

These results demonstrate that the compound and antioxidant of the present invention has LO• scavenging ability and LOO• scavenging ability and also is superior to methyldopa in terms of a balance between the LO• scavenging ability and the LOO• scavenging ability. Also, it is known that compounds with LO• scavenging ability LOO• scavenging ability are capable of protecting cells from radicals. Accordingly, the compound of the present invention is expected to be extremely excellent not only as an antioxidant but also as a cell protectant.

Example 3

The present example was performed in order to confirm whether the compound of the present invention can effectively suppress ferroptosis and whether the compound of the present invention has ability to promote cell growth.

Mouse hippocampal neurons (HT-22, Merck) were seeded in a 96-well, flat-bottom plate at a density of $5.0 \times 10^3$ cells/100 μl/well and cultured for 24 hours. The neurons were passaged four times after the purchase. The culture medium used in the above culture was DMEM (High Glucose, Nacalai Tesque) containing 10% fetal bovine serum (Nacalai Tesque) and 1 × penicillin/streptomycin (Nacalai Tesque). The culture conditions were set to wet environment at 37°C and 5% $CO_2$.

After the culture, a sample solution containing the compound represented by any one of the formulae (3) to (5) or methyldopa and ferroptosis inducers (elastin (Cayman Chemical Company) and RSL-3 (Sigma-Aldrich)) was added at 100 μl/well. Each of the compounds represented by the formulae (3) to (5) or the methyldopa was added so as to achieve a predetermined final concentration in each well (0 (control), 1, 3, or 10 μmol/l). The ferroptosis inducers were added so as to achieve predetermined final concentrations in each well, respectively (elastin: 0.2 μmol/l; RSL-3: 0.05 μmol/l). After adding these components, an MTT reagent (Nacalai Tesque) was added at 15 μl/well, and the neurons were cultured for 2 hours under the above-described culture conditions.

Subsequently, the medium was aspirated from each well, and DMSO was added at 100 μl/well. Thereafter, the absorbance at 570 nm and the absorbance at 650 nm of each well were measured using an EnSpire plate reader (PerkinElmer). The cell-viability index of each well was determined using the absorbance of a formazan dye (570 nm) and the background absorbance (650 nm). For the control group, the cell-viability index was determined in the same manner, except that the compound represented by any one of the formulae (3) to (5) or the methyldopa and the ferroptosis inducers was not added. Then the relative value of the cell viability of each drug-treated group was calculated with the index of the control as 100% cell viability. The results obtained are shown in FIG. 5.

FIG. 5 shows graphs showing the cell viability. In FIG. 5, the horizontal axis indicates the type of the sample or the concentration of the compound represented by any of the formulae (3) to (5) or the methyldopa, and the vertical axis indicates the cell viability. In each of the graphs, bars indicate, from left, the result obtained regarding the control (ctrl) and the results obtained when the concentration of the compound represented by any of the formulae (3) to (5) or the methyldopa was 0 (-), 1, 3, or 10 μmol/l, respectively. As can be seen from FIG. 5, the compounds represented by the formulae (3) to (5) suppressed ferroptosis in a concentration-dependent manner. Also, the compounds represented by the formulae (3) to (5) achieved a significantly higher degree of suppression of ferroptosis than methyldopa. As described above, the compounds represented by the formulae (3) to (5) are superior to methyldopa in terms of a balance between the LO• scavenging ability and the LOO• scavenging ability. Accordingly, it is presumed that the compounds represented by the formulae (3) to (5) exhibited their cell-protective function by effectively scavenging these radicals, which constitute a factor causing ferroptosis.

Also, as can be seen from FIG. 5, in the group in which the compounds represented by the formulae (3) to (5) were at a concentration of 10 μmol/l, the cell viabilities were all improved as compared with that in the control group. In other words, these compounds improved the cell growth. From these results, it was found that the compound of the present invention has ability to promote cell growth.

The above results demonstrate that the compound of the present invention can effectively suppress ferroptosis and also that the compound of the present invention has ability to promote cell growth.

Example 4

The present example was performed in order to confirm whether the compound of the present invention has ability to promote cell growth.

In the same manner as in Example 3, the same mouse hippocampal neurons were seeded in a 96-well, flat-bottom plate and cultured for 24 or 48 hours. Before culture, a sample solution containing the compound represented by any one of the formulae (3) to (5) was added at 100 μl/well. Each of the compounds represented by the formulae (3) to (5) was added so as to achieve a predetermined final concentration in each well (0 (control), 3, or 10 μmol/l). After the culture, an MTT reagent (Nacalai Tesque) was added at 15 μl/well, and the neurons were cultured for 2 hours under the above-described culture conditions.

For each well after the culture, the cell-viability index was determined in the same manner as in Example 3. Then the relative value of the cell viability of each drug-treated group was calculated with the index of the control as 100% cell viability. The results obtained are shown in FIGs. 6A and 6B.

FIGs. 6A and 6B are graphs showing the cell viability. In FIGs. 6A and 6B, the horizontal axis indicates the type of the sample or the concentration of the compound represented by any one of the formulae (3) to (5), and the vertical axis indicates the cell viability. FIG. 6A shows the results obtained after 24 hours of culture, and FIG. 6B shows the results obtained after 48 hours of culture. As can be seen from FIGs. 6A and 6B, the cell viabilities when the compounds represented by the formulae (3) to (5) were used were all improved as compared with those in the control group. In other words, these compounds improved cell growth. These results confirm that the compound of the present invention has ability to promote cell growth. As described above, the compound of the present invention functions as an antioxidant. Accordingly, it is presumed that the compound of the present invention promotes cell growth by exhibiting an antioxidative function in cells.

While the present invention has been described above with reference to exemplary embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application Nos. 2020-023164 and 2020-023221 filed on February 14, 2020. All of the disclosures of these Japanese patent applications are incorporated herein by reference.

SUPPLEMENTARY NOTES

Part or the whole of the exemplary embodiments and examples disclosed above can be described as in the following supplementary notes. It is to be noted, however, that the present invention is by no means limited thereto.

(Supplementary Note 1)

A compound represented by the following formula (1) or a salt thereof:

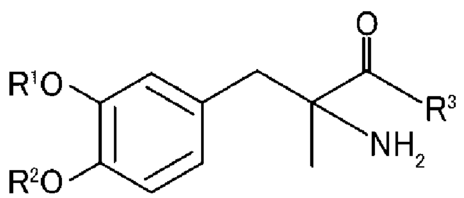

···(1)

where, in the formula (1),

- $R^1$ and $R^2$ may be the same or different and are each independently a hydrogen atom, an alkyl group, or an acyl group,
- $R^3$ is $-OR^4$ or $-NHR^5$,
- $R^4$ is a sec-butyl group, a tert-butyl group, or an iso-butyl group, and
- $R^5$ is a sec-butyl group, a tert-butyl group, or an iso-butyl group.

(Supplementary Note 2)

The compound or salt thereof according to Supplementary Note 1, wherein the compound is represented by the following formula (2):

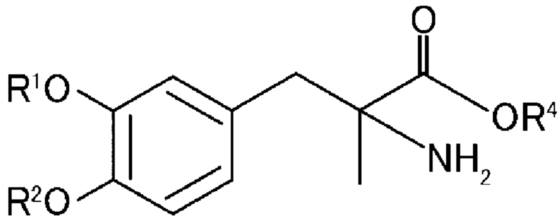

···(2)

where, in the formula (2),

$R^1$ and $R^2$ may be the same or different and are each independently a hydrogen atom, an alkyl group, or an acyl group, and
$R^4$ is a sec-butyl group, a tert-butyl group, or an iso-butyl group.

(Supplementary Note 3)

The compound or salt thereof according to Supplementary Note 1 or 2, wherein the compound is represented by the following formula (3):

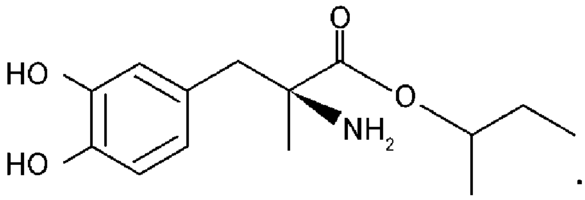

···(3)

(Supplementary Note 4)

The compound or salt thereof according to Supplementary Note 1 or 2, wherein the compound is represented by the following formula (4):

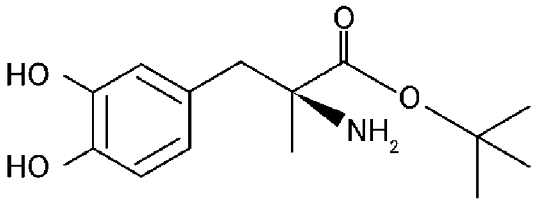

···(4)

(Supplementary Note 5)

The compound or salt thereof according to Supplementary Note 1 or 2, wherein the compound is represented by the following formula (5):

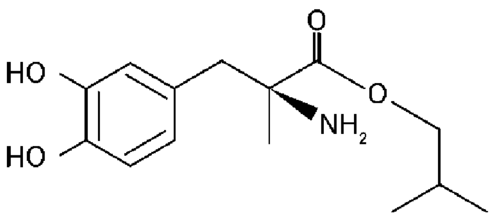

···(5)

(Supplementary Note 6)

The compound or salt thereof according to Supplementary Note 1, wherein the compound is represented by the following formula (6):

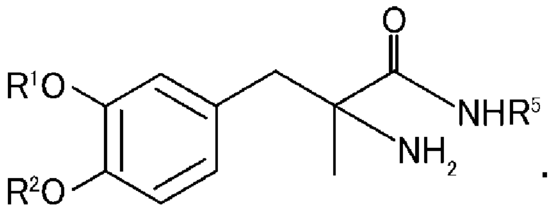

...(6)

where, in the formula (6),

$R^1$ and $R^2$ may be the same or different and are each independently a hydrogen atom, an alkyl group, or an acyl group, and
$R^5$ is a sec-butyl group, a tert-butyl group, or an iso-butyl group.

(Supplementary Note 7)

A pharmaceutical composition containing:

the compound or salt thereof according to any one of Supplementary Notes 1 to 6; and
a pharmaceutically acceptable carrier.

(Supplementary Note 8)

An antioxidant containing:
a compound represented by the following formula (1A) or a salt thereof:

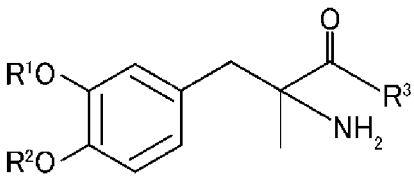

...(1A)

where, in the formula (1A),

$R^1$ and $R^2$ may be the same or different and are each independently a hydrogen atom, an alkyl group, or an acyl group,
$R^3$ is $-OR^4$ or $NHR^5$,
$R^4$ is an alkyl group, and
$R^5$ is a hydrogen atom or an alkyl group.

(Supplementary Note 9)

The antioxidant according to Supplementary Note 8, wherein the compound is represented by the following formula (2):

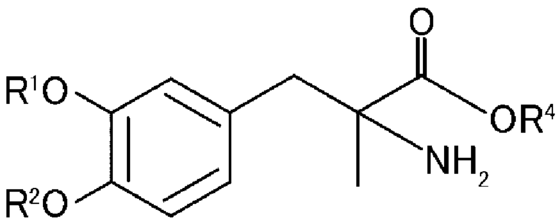

...(2)

where, in the formula (2),

$R^1$ and $R^2$ may be the same or different and are each independently a hydrogen atom, an alkyl group, or an acyl group, and
$R^4$ is an alkyl group.

(Supplementary Note 10)

The antioxidant according to Supplementary Note 8 or 9, wherein the compound is represented by the following formula (3):

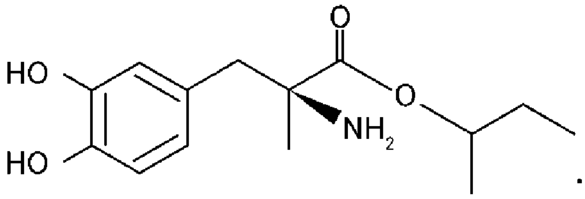

···(3)

(Supplementary Note 11)

The antioxidant according to Supplementary Note 8 or 9, wherein the compound is represented by the following formula (4):

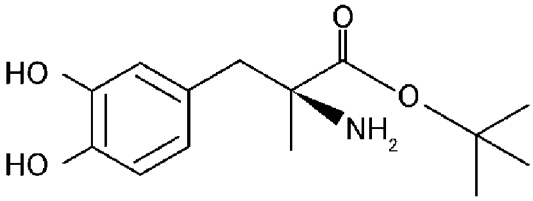

···(4)

(Supplementary Note 12)

The antioxidant according to Supplementary Note 8 or 9, wherein the compound is represented by the following formula (5):

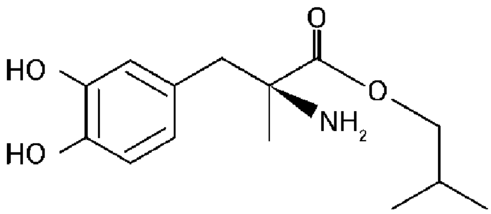

···(5)

(Supplementary Note 13)

The antioxidant according to Supplementary Note 8 or 9, wherein the compound is represented by the following formula (7):

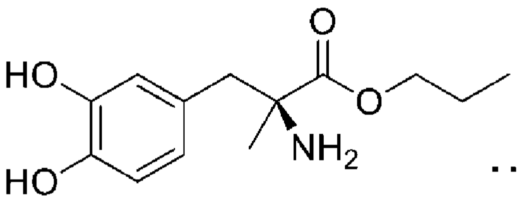

···(7)

(Supplementary Note 14)

The antioxidant according to Supplementary Note 8 or 9, wherein the compound is represented by the following formula (8):

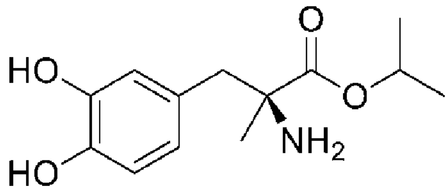

...(8)

(Supplementary Note 15)

The antioxidant according to Supplementary Note 8 or 9, wherein the compound is represented by the following formula (9):

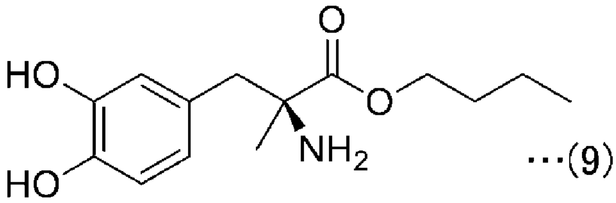

...(9)

(Supplementary Note 16)

The antioxidant according to Supplementary Note 8 or 9, wherein the compound is represented by the following formula (10):

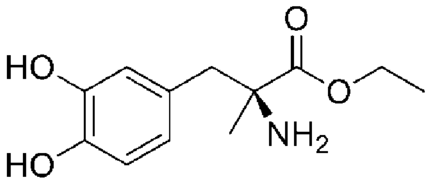

...(10)

(Supplementary Note 17)

The antioxidant according to Supplementary Note 8, wherein the compound is represented by the following formula (6):

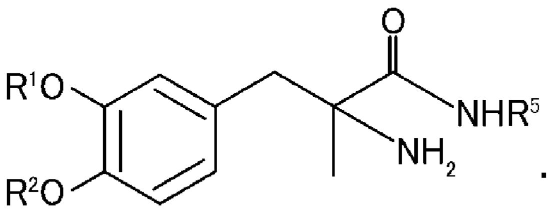

...(6)

where, in the formula (6),

$R^1$ and $R^2$ may be the same or different and are each independently a hydrogen atom, an alkyl group, or an acyl group, and
$R^5$ is a hydrogen atom or an alkyl group.

(Supplementary Note 18)

A cell protectant containing:
the compound or salt thereof according to any one of Supplementary Notes 1 to 6 or the antioxidant according to any one of Supplementary Notes 8 to 17.

(Supplementary Note 19)

A cell growth-promoting agent containing:
the compound or salt thereof according to any one of Supplementary Notes 1 to 6 or the antioxidant according to any one of Supplementary Notes 8 to 17.

(Supplementary Note 20)

A method for preventing oxidation using the antioxidant according to any one of Supplementary Notes 8 to 17.

(Supplementary Note 21)

The method according to Supplementary Note 20, including the step of:
bringing a target substance and the antioxidant into contact with each other.

(Supplementary Note 22)

A method for protecting a cell using the cell protectant according to Supplementary Note 18.

(Supplementary Note 23)

The method according to Supplementary Note 22, wherein the cell and the cell protectant are caused to be present together.

(Supplementary Note 24)

A method for promoting cell growth using the cell growth-promoting agent according to Supplementary Note 19.

(Supplementary Note 25)

The method according to Supplementary Note 24, wherein a cell and the cell growth-promoting agent are caused to be present together.

(Supplementary Note 26)

Use of the compound represented by the formula (1) or (1A) or a salt thereof for anti oxidation.

(Supplementary Note 27)

Use of the compound represented by the formula (1) or (1A) or a salt thereof for cell protection.

(Supplementary Note 28)

Use of the compound represented by the formula (1) or (1A) or a salt thereof for promotion of cell growth.

INDUSTRIAL APPLICABILITY

As described above, the compound of the present invention or a salt thereof is superior to methyldopa in terms of achieving a balance between the LO• scavenging ability and the LOO• scavenging ability. Accordingly, the compound of the present invention is expected to be applicable to the treatment of diseases caused by radicals, such as diseases for which edaravone is used for treatment, for example. Therefore, it is considered that the present invention is very useful in the field of pharmaceuticals and the like.

## Claims

1. A compound represented by the following formula (1) or a salt thereof:

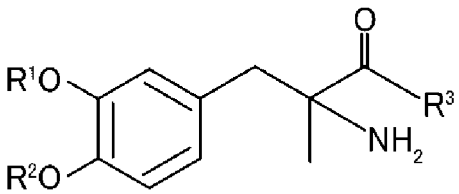

...(1)

where, in the formula (1),

$R^1$ and $R^2$ may be the same or different and are each independently a hydrogen atom, an alkyl group, or an acyl group,
$R^3$ is $-OR^4$ or $-NHR^5$,
$R^4$ is a sec-butyl group, a tert-butyl group, or an iso-butyl group, and
$R^5$ is a sec-butyl group, a tert-butyl group, or an iso-butyl group.

2. The compound or salt thereof according to claim 1, wherein the compound is represented by the following formula (2):

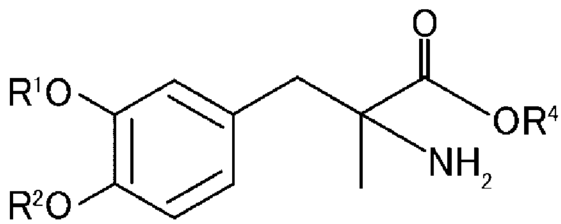

...(2)

where, in the formula (2),

$R^1$ and $R^2$ may be the same or different and are each independently a hydrogen atom, an alkyl group, or an acyl group, and
$R^4$ is a sec-butyl group, a tert-butyl group, or an iso-butyl group.

3. The compound or salt thereof according to claim 1 or 2, wherein the compound is represented by the following formula (3):

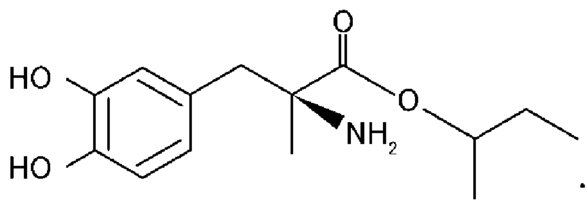

...(3)

4. The compound or salt thereof according to claim 1 or 2, wherein the compound is represented by the following formula (4):

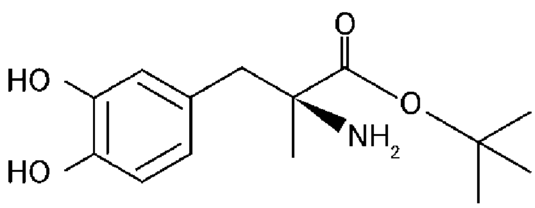

...(4)

5. The compound or salt thereof according to claim 1 or 2, wherein the compound is represented by the following formula (5):

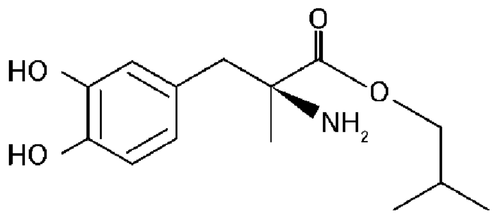

...(5)

6. The compound or salt thereof according to claim 1, wherein the compound is represented by the following formula (6):

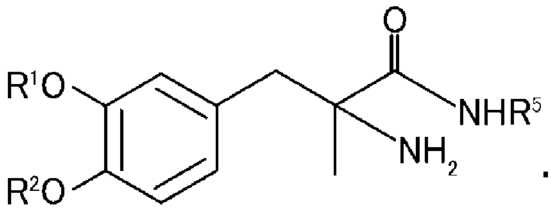

...(6)

where, in the formula (6),

$R^1$ and $R^2$ may be the same or different and are each independently a hydrogen atom, an alkyl group, or an acyl group, and
$R^5$ is a sec-butyl group, a tert-butyl group, or an iso-butyl group.

7. A pharmaceutical composition comprising:

the compound or salt thereof according to any one of claims 1 to 6; and
a pharmaceutically acceptable carrier.

8. An antioxidant comprising:
a compound represented by the following formula (1A) or a salt thereof:

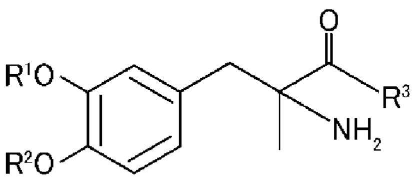

...(1A)

where, in the formula (1A),

$R^1$ and $R^2$ may be the same or different and are each independently a hydrogen atom, an alkyl group, or an acyl group,
$R^3$ is $-OR^4$ or $NHR^5$,
$R^4$ is an alkyl group, and
$R^5$ is a hydrogen atom or an alkyl group.

9. The antioxidant according to claim 8, wherein the compound is represented by the following formula (2):

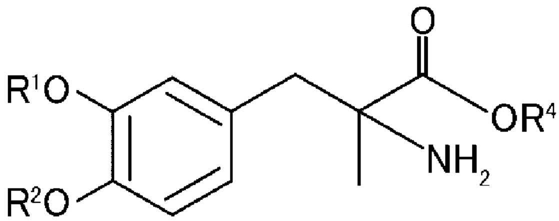

…(2)

where, in the formula (2),

$R^1$ and $R^2$ may be the same or different and are each independently a hydrogen atom, an alkyl group, or an acyl group, and
$R^4$ is an alkyl group.

**10.** The antioxidant according to claim 8 or 9, wherein the compound is represented by the following formula (3):

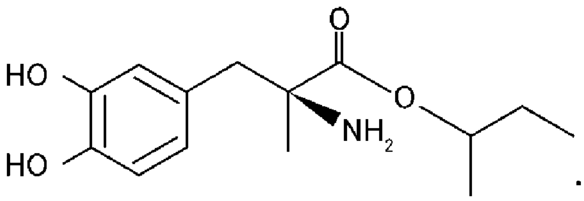

…(3)

**11.** The antioxidant according to claim 8 or 9, wherein the compound is represented by the following formula (4):

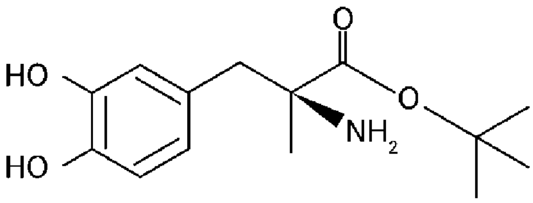

…(4)

**12.** The antioxidant according to claim 8 or 9, wherein the compound is represented by the following formula (5):

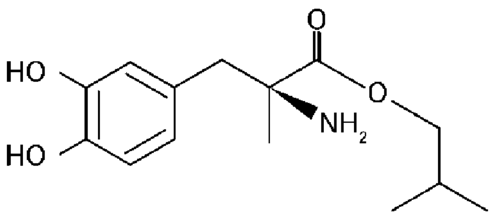

…(5)

**13.** The antioxidant according to claim 8 or 9, wherein the compound is represented by the following formula (7):

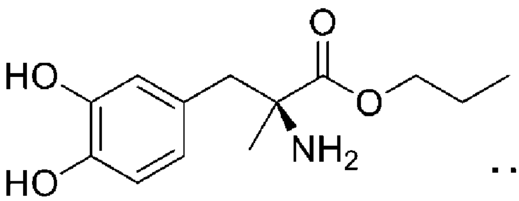

…(7)

**14.** The antioxidant according to claim 8 or 9, wherein the compound is represented by the following formula (8):

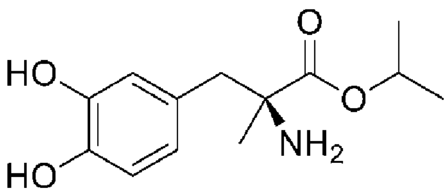

...(8)

**15.** The antioxidant according to claim 8 or 9, wherein the compound is represented by the following formula (9):

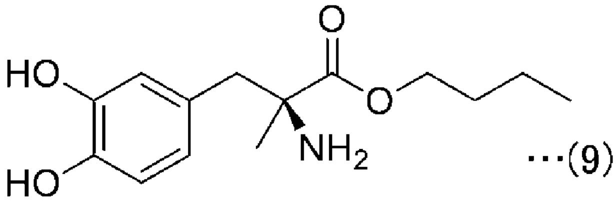

...(9)

**16.** The antioxidant according to claim 8 or 9, wherein the compound is represented by the following formula (10):

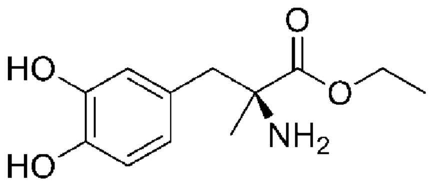

...(10)

**17.** The antioxidant according to claim 8, wherein the compound is represented by the following formula (6):

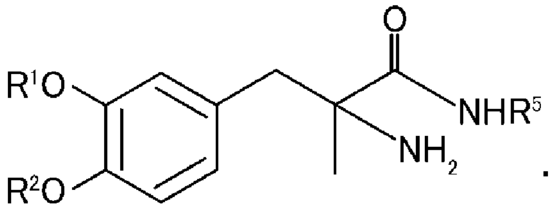

...(6)

where, in the formula (6),

$R^1$ and $R^2$ may be the same or different and are each independently a hydrogen atom, an alkyl group, or an acyl group, and
$R^5$ is a hydrogen atom or an alkyl group.

**18.** A cell protectant comprising:
the compound or salt thereof according to any one of claims 1 to 6 or the antioxidant according to any one of claims 8 to 17.

**19.** A cell growth-promoting agent comprising:
the compound or salt thereof according to any one of claims 1 to 6 or the antioxidant according to any one of claims 8 to 17.

**20.** A method for preventing oxidation using the antioxidant according to any one of claims 8 to 17.

**21.** The method according to claim 20, comprising the step of:
bringing a target substance and the antioxidant into contact with each other.

**22.** A method for protecting a cell by using the cell protectant according to claim 18.

**23.** The method according to claim 22, wherein the cell and the cell protectant are caused to be present together.

**24.** A method for promoting cell growth by using the cell growth-promoting agent according to claim 19.

**25.** The method according to claim 24, wherein a cell and the cell growth-promoting agent are caused to be present together.

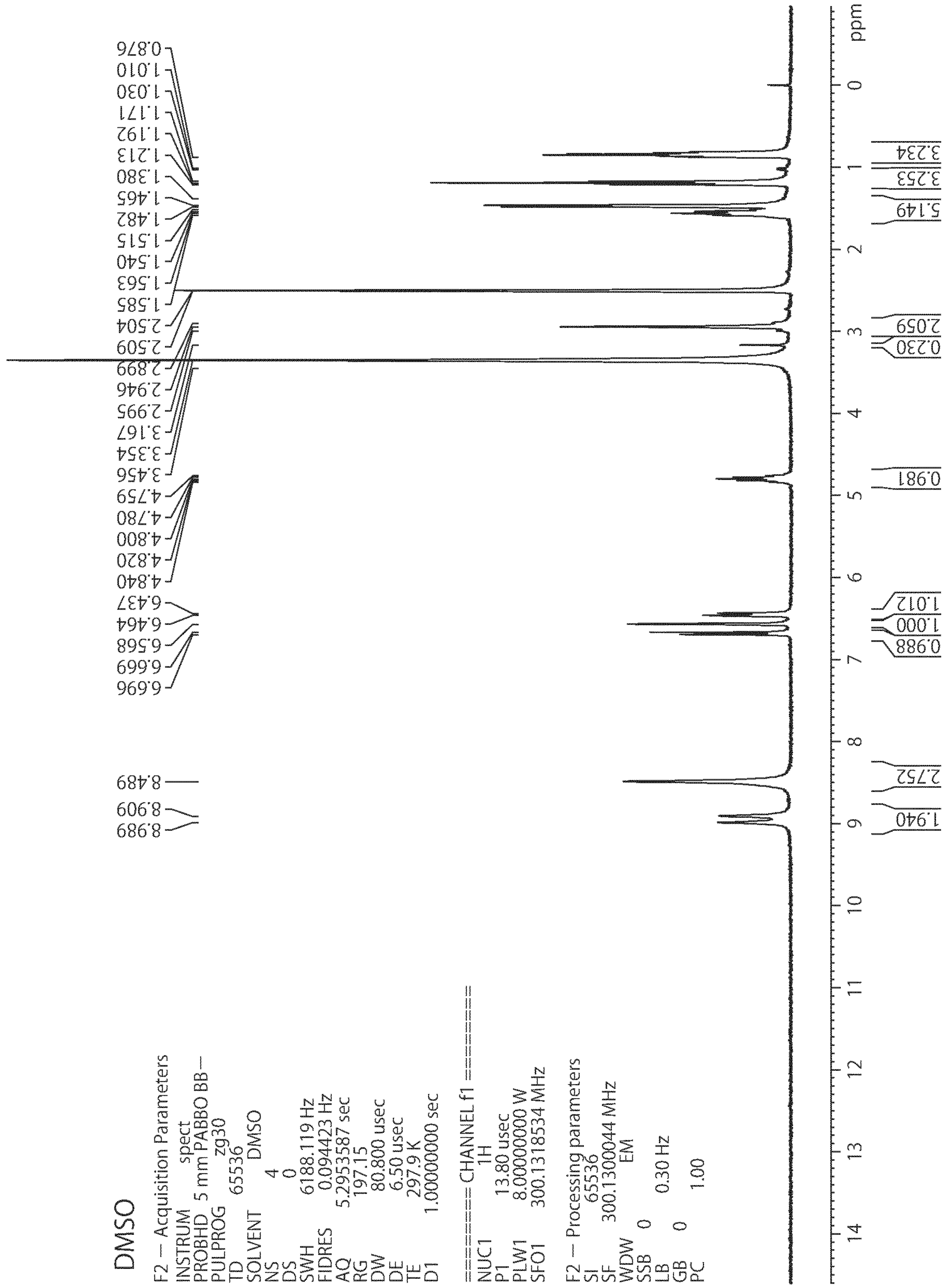
DMSO
F2 — Acquisition Parameters
INSTRUM spect
PROBHD 5 mm PABBO BB—
PULPROG zg30
TD 65536
SOLVENT DMSO
NS 4
DS 0
SWH 6188.119 Hz
FIDRES 0.094423 Hz
AQ 5.2953587 sec
RG 197.15
DW 80.800 usec
DE 6.50 usec
TE 297.9 K
D1 1.00000000 sec
======== CHANNEL f1 ========
NUC1 1H
P1 13.80 usec
PLW1 8.00000000 W
SFO1 300.1318534 MHz
F2 — Processing parameters
SI 65536
SF 300.1300044 MHz
WDW EM
SSB 0
LB 0.30 Hz
GB 0
PC 1.00
0.876
1.010
1.030
1.171
1.192
1.213
1.380
1.465
1.482
1.515
1.540
1.563
1.585
2.504
2.509
2.899
2.946
2.995
3.167
3.354
3.456
4.759
4.780
4.800
4.820
4.840
6.437
6.464
6.568
6.669
6.696
8.489
8.909
8.989
3.234
3.253
5.149
2.059
0.230
0.981
1.012
1.000
0.988
2.752
1.940
ppm
0
1
2
3
4
5
6
7
8
9
10
11
12
13
14

FIG. 1

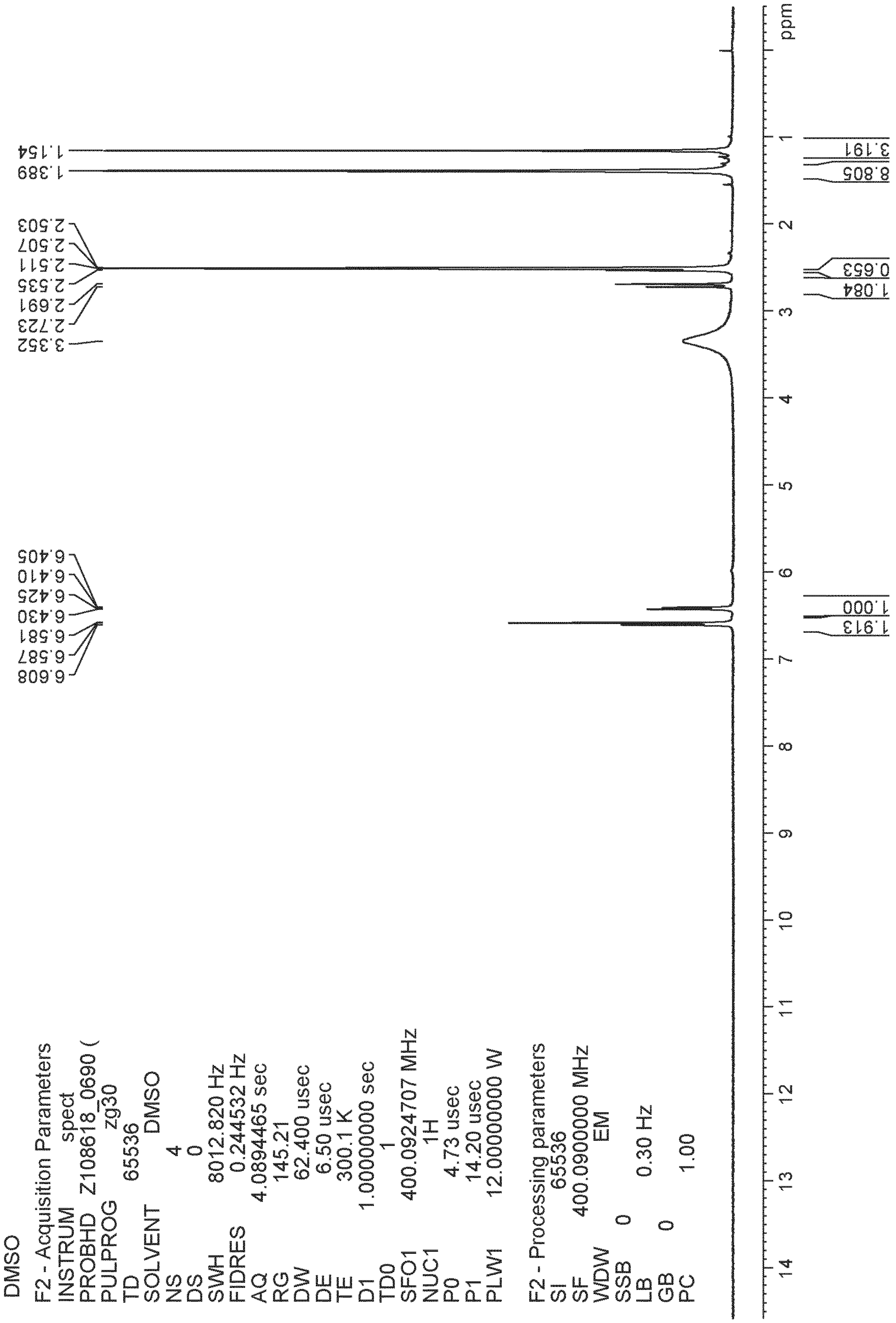
DMSO
F2 - Acquisition Parameters
INSTRUM spect
PROBHD Z108618_0690 (
PULPROG zg30
TD 65536
SOLVENT DMSO
NS 4
DS 0
SWH 8012.820 Hz
FIDRES 0.244532 Hz
AQ 4.0894465 sec
RG 145.21
DW 62.400 usec
DE 6.50 usec
TE 300.1 K
D1 1.00000000 sec
TD0 1
SFO1 400.0924707 MHz
NUC1 1H
P0 4.73 usec
P1 14.20 usec
PLW1 12.00000000 W
F2 - Processing parameters
SI 65536
SF 400.0900000 MHz
WDW EM
SSB 0
LB 0.30 Hz
GB 0
PC 1.00
1.154
1.389
2.503
2.507
2.511
2.535
2.691
2.723
3.352
6.405
6.410
6.425
6.430
6.581
6.587
6.608
3.191
8.805
0.653
1.084
1.000
1.913
ppm
1
2
3
4
5
6
7
8
9
10
11
12
13
14

FIG. 2

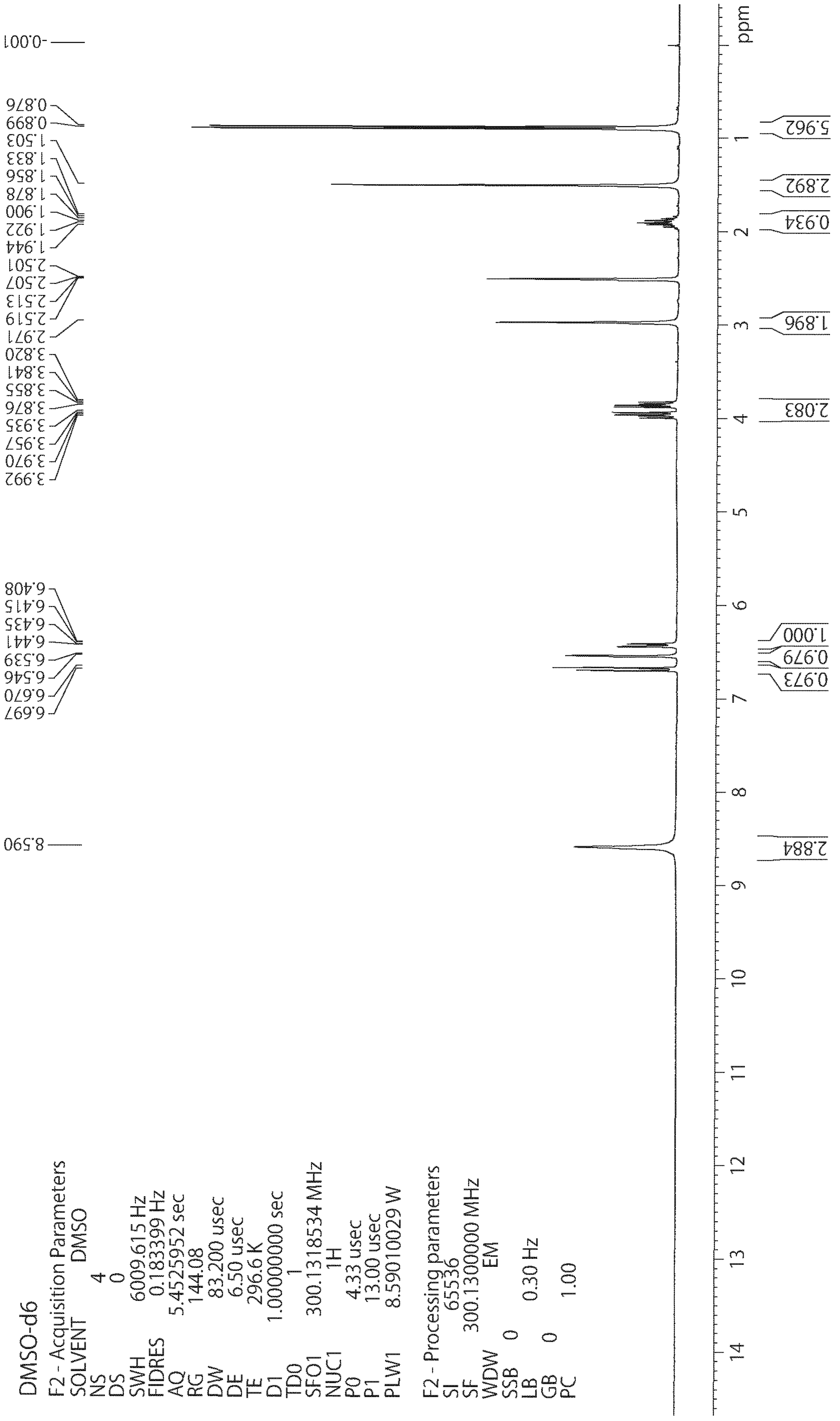
-0.001
0.876
0.899
1.503
1.833
1.856
1.878
1.900
1.922
1.944
2.501
2.507
2.513
2.519
2.971
3.820
3.841
3.855
3.876
3.935
3.957
3.970
3.992
6.408
6.415
6.435
6.441
6.539
6.546
6.670
6.697
8.590
5.962
2.892
0.934
1.896
2.083
1.000
0.979
0.973
2.884
ppm
DMSO-d6
F2 - Acquisition Parameters
SOLVENT DMSO
NS 4
DS 0
SWH 6009.615 Hz
FIDRES 0.183399 Hz
AQ 5.4525952 sec
RG 144.08
DW 83.200 usec
DE 6.50 usec
TE 296.6 K
D1 1.00000000 sec
TD0 1
SFO1 300.1318534 MHz
NUC1 1H
P0 4.33 usec
P1 13.00 usec
PLW1 8.59010029 W
F2 - Processing parameters
SI 65536
SF 300.1300000 MHz
WDW EM
SSB 0
LB 0.30 Hz
GB 0
PC 1.00

FIG. 3

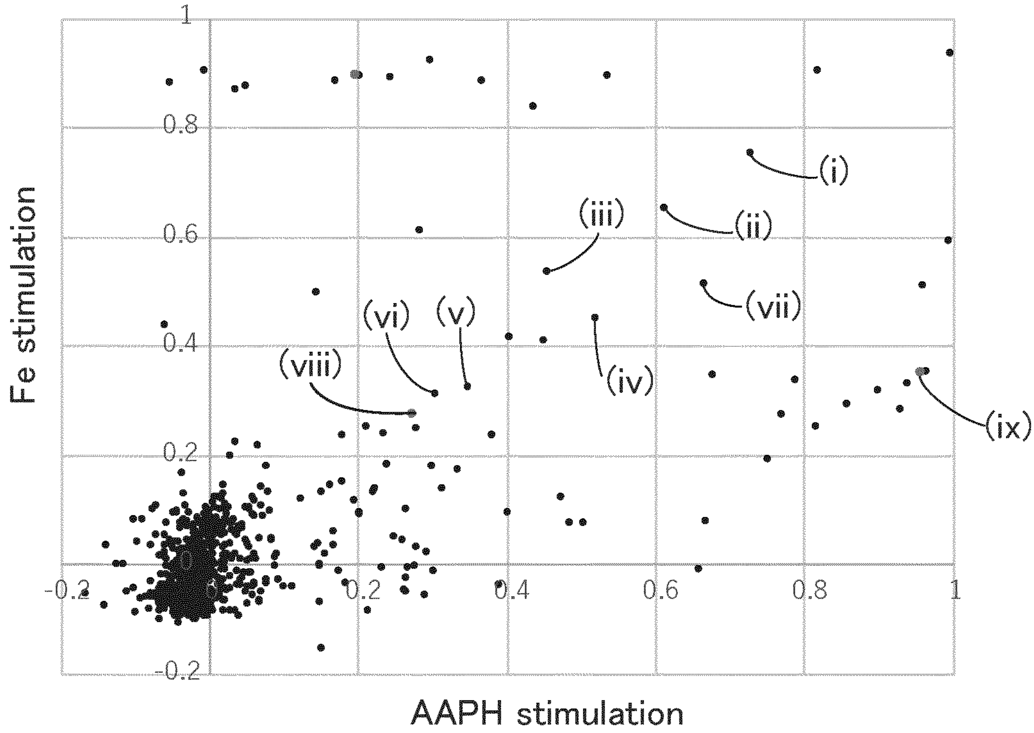


(i) Compound of formula (3)
(ii) Compound of formula (4)
(iii) Compound of formula (5)
(iv) Compound of formula (7)
(v) Compound of formula (8)
(vi) Compound of formula (9)
(vii) Compound offormula (10)
(viii) Edaravone
(ix) Methyldopa(L-isomer)

FIG. 4

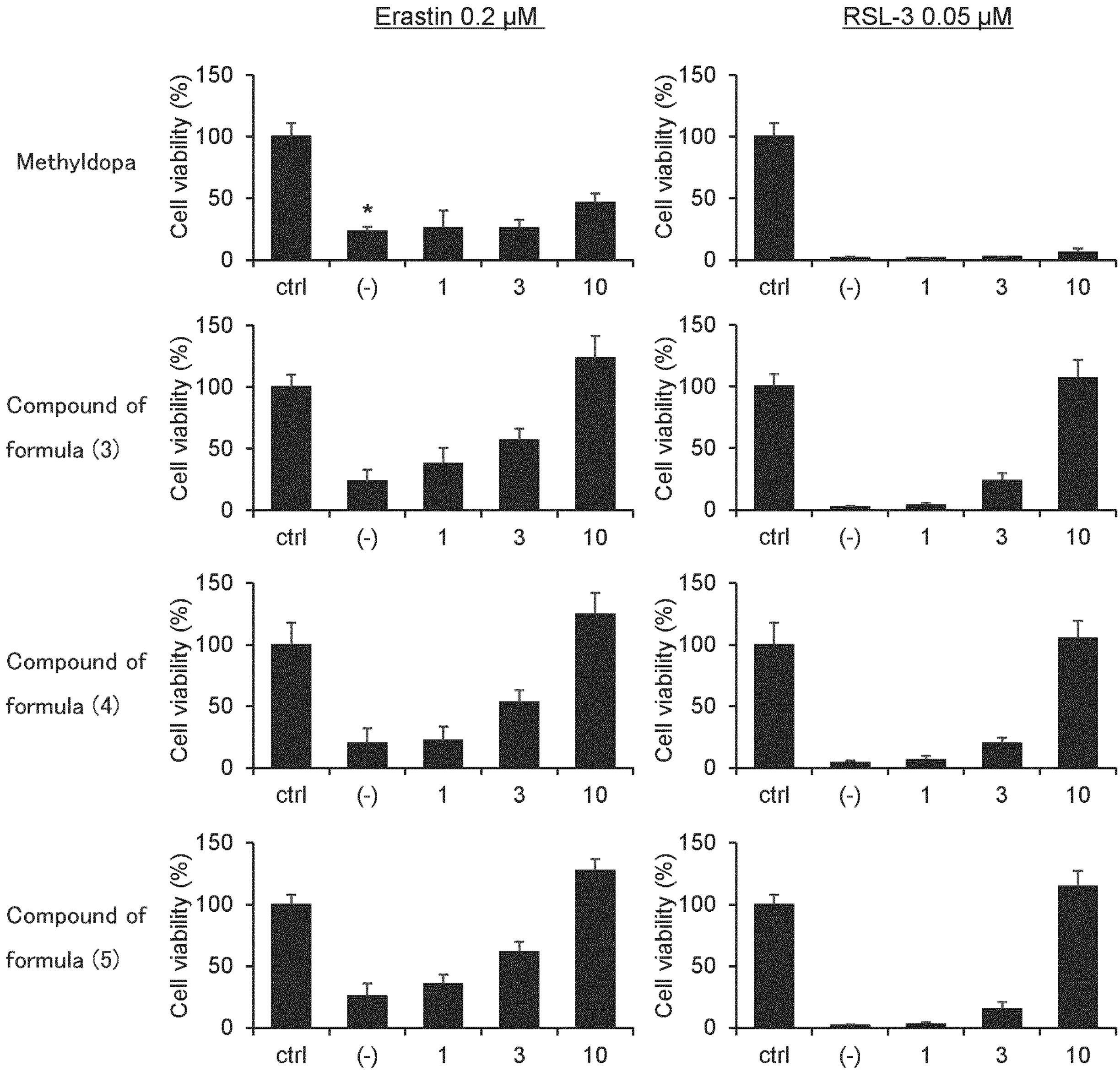


Mean + S.D., (n=6), *p<0.05 v.s. ctrl, #p<0.05 v.s. (-)

FIG. 5

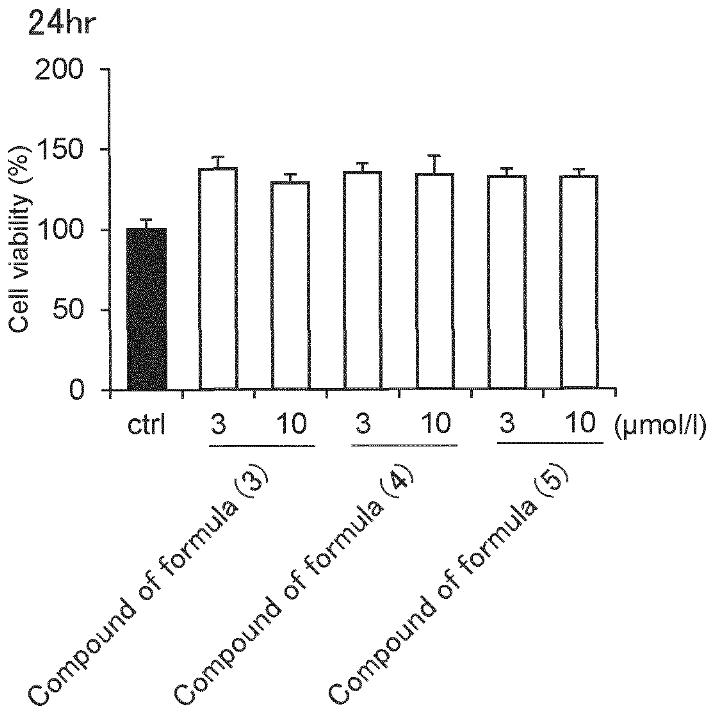
24hr
200
150
100
50
0
Cell viability (%)
ctrl
3
10
3
10
3
10
(μmol/l)
Compound of formula (3)
Compound of formula (4)
Compound of formula (5)

FIG. 6A

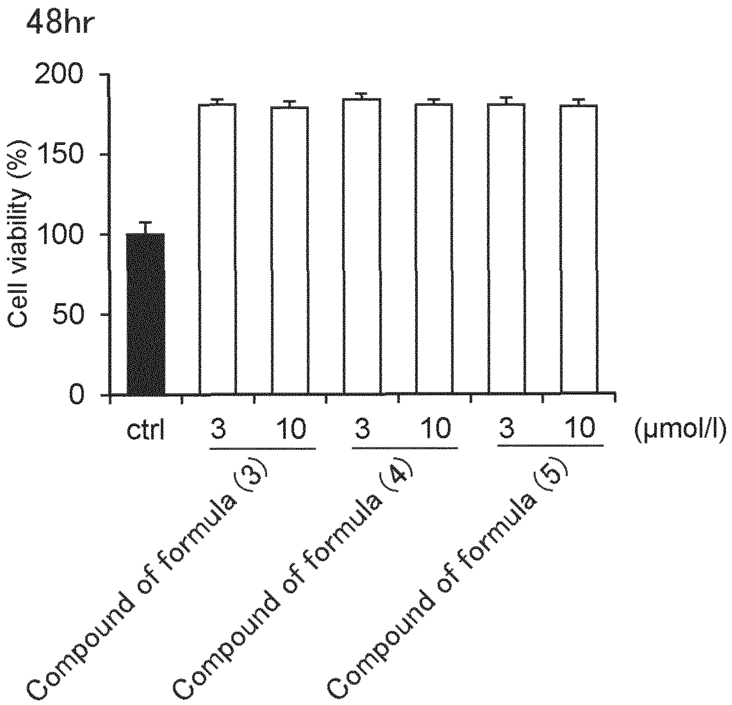
48hr
200
150
100
50
0
Cell viability (%)
ctrl
3
10
3
10
3
10
(μmol/l)
Compound of formula (3)
Compound of formula (4)
Compound of formula (5)

FIG. 6B

**INTERNATIONAL SEARCH REPORT**

International application No. PCT/JP2021/005302

A. CLASSIFICATION OF SUBJECT MATTER
Int.Cl. C07C229/36(2006.01)i, A61K31/198(2006.01)i, A61K31/223(2006.01)i, A61P9/00(2006.01)i, A61P11/00(2006.01)i, A61P25/00(2006.01)i, A61P31/04(2006.01)i, A61P39/06(2006.01)i, A61P43/00(2006.01)i
FI: C07C229/36CSP, A61K31/198, A61P39/06, A61P43/00105, A61P9/00, A61P25/00, A61P11/00, A61P31/04, A61K31/223
According to International Patent Classification (IPC) or to both national classification and IPC

B. FIELDS SEARCHED
Minimum documentation searched (classification system followed by classification symbols)
Int.Cl. C07C229/36, A61K31/198, A61K31/223, A61P9/00, A61P11/00, A61P25/00, A61P31/04, A61P39/06, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | OGURI, T. et al., Amino acids and peptides. XXVIII. A new synthesis of α-amino acid derivatives by alkylation of schiff bases derived from glycine and alanine, Chem. Pharm. Bull., 1977, vol. 25, no. 9, pp. 2287-2291, p. 2288, chart 1, compound S-Vb | 1, 2, 4<br>3, 5-25 |
| X<br>A | Databese REGISTRY, 08 July 2019, STN international [online], [retrieved on 18 March 2020], RN:2352975-47-2, entire text, CAS registration number: 2352975-47-2, compound | 1, 2, 4<br>3, 5-25 |
| X<br>A | Databese REGISTRY, 15 March 2019, STN international [online], [retrieved on 18 March 2020], RN: 2287247-96-3, entire text, CAS registration number: 2287247-96-3, compound | 1, 2, 4<br>3, 5-25 |

☒ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>18 March 2021 | Date of mailing of the international search report<br>30 March 2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/005302

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Database REGISTRY, 10 March 2014, STN international [online], [retrieved on 18 March 2020], RN: 1565206-30-5, entire text, CAS registration number: 1565206-30-5, compound | 1, 5, 6<br>2-4, 7-25 |
| X<br>A | Databese REGISTRY, 09 July 2019, STN international [online], [retrieved on 18 March 2020], RN: 2354426-71-2, entire text, CAS registration number: 2354426-71-2, compound | 1, 2<br>3-25 |
| X<br>A | Databese REGISTRY, 09 July 2019, STN international [online], [retrieved on 18 March 2020], RN2354426-58-5, entire text, CAS registration number: 2354426-58-5, compound | 1, 2<br>3-25 |
| X<br>A | Databese REGISTRY, 15 July 2019, STN international [online], [retrieved on 18 March 2020], RN:2358591-41-8, entire text, CAS registration number: 2358591-41-8, compound | 1, 2<br>3-25 |
| A | GB 936074 A (MERCK & CO., INC.) 04 September 1963 (1963-09-04), entire text, all drawings, particularly, page 3, scheme 2, example 15 | 1-25 |
| A | WO 2019/009355 A1 (YAMADA, Kenichi) 10 January 2019 (2019-01-10), entire text, all drawings, particularly, compound Y, examples | 1-25 |

Form PCT/ISA/210 (second sheet) (January 2015)

**INTERNATIONAL SEARCH REPORT**
Information on patent family members

International application No.
PCT/JP2021/005302

| | |
|---|---|
| GB 936074 A 04 September 1963 | US 3344023 A<br>entire text, all drawings, particularly, column 3, III-amino-nitrile synthesis, example 15 |
| WO 2019/009355 A1 10 January 2019 | US 2020/0158709 A1<br>entire text, all drawings, particularly, compound Y, examples<br>CN 111033257 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019009355 A
- JP 2020023164 A
- JP 2020023221 A


### Non-patent literature cited in the description


- **JEROEN FRIJHOFF et al.** Clinical Relevance of Biomarkers of Oxidative Stress. *Antioxidants & Redox Signaling,* vol. 23 (14), 1144-1170